# EUROPEAN PATENT APPLICATION

(11) **EP 1 083 231 A1**
(43) Date of publication of application: **14.03.2001**
(21) Application number: 99202943.9
(22) Date of filing: 09.09.1999
(51) Int. Cl.: C12N 15/861, C12N 15/53, C12N 15/12, C12N 5/10, C07K 14/47, A61K 48/00, A61P 9/10, C12Q 1/68

(54) **Smooth muscle cell promoter and uses thereof**

(71) Applicant: Introgene B.V., 2333 AL Leiden (NL)
(72) Inventor: The designation of the inventor has not yet been filed
(74) Representative: Ottevangers, Sietse Ulbe

(57) **Abstract**

The present invention discloses novel smooth muscle cell specific promoters and uses thereof. The promoters are suited for providing nucleic acid of interest with the capability of expressing specifically in a contractile smooth muscle cell, preferably a vascular contractile smooth muscle cell and/or a visceral contractile smooth muscle cell. One example of such a promoter is a smoothelin gene promoter. For gene therapy purposes said smoothelin gene promoter is preferably derived from a human. A promoter of the invention may be incorporated into nucleic acid delivery vehicle. The nucleic acid delivery vehicle preferably comprises a virus-like particle such as an adenovirus particle, an adeno-associated virus particle or a retrovirus particle.

## Description

The invention relates to the fields of medicine, molecular biology and especially to gene therapy. More particularly, the invention relates to means and methods for obtaining tissue specific expression.

Smooth muscle cells (SMC) are a collection of cells with a number of common characteristics. They share the expression of SMC-specific proteins such as calponin, α-smooth muscle actin and smooth muscle myosin. On the other hand they lack proteins characteristic for striated muscle cells, such as titin and nebulin. However, amongst SMCs, differences have been observed concerning morphology, embryological origin, state of differentiation and anatomical position (Gabbiani et al., 1981,; Haeberle et al., 1992; Owens, 1995). Therefore, SMCs can be divided in subpopulations, for instance SMCs of a proliferative and contractile phenotype (Campbell and Chamley-Campbell, 1981; Skalli et al., 1986; Owens, 1995). Also, SMCs of the visceral organs, digestive tract and the reproductive system, have been shown to differ from the SMCs found in the blood vessels. Smooth muscle cells aligning the digestive tract and ducts associated with the gut, SMCs around bladder, trachea and bronchi are all derived of the lateral mesoderm (Gilbert, 1991). The available evidence on the embryological origin of vascular SMCs indicate that the majority of these cells is derived locally. The premordium, invaded by endothelial cells, contributes the SMCs to the developing vessel (Christ and Ordahl, 1995; Yablonka-Reuveni et al, 1993). In addition, recent data indicate a possible transition from endothelial to SMC (De Ruiter et al., 1997). The heterogeneity in origin, morphology and function has been hampering the elucidation of the molecular mechanisms involved in differentiation of SMCs.

SMCs are the most abundant cell type in tissues of the digestive tract, the urogenital system and in blood vessel walls. They occur in all vessels except capillaries and pericytic venules. In the media of the vascular wall, SMCs are intermingled with elastic lamellae and laminae, and appear as concentric, helically arranged bundles. Amongst SMCs considerable variations in morphology and physiology have been found. This may be due to differences in embryologic origin and differentiation (Gabbiani et al. 1981; Haeberle et al. 1992; Owens 1995). Roughly, SMCs can be divided in two sub-populations, e.g. proliferative (or synthetic) and contractile phenotypes, which should be considered as the extremes of a scale (Owens 1995; Campbell et al. 1989; Skalli et al. 1986). SMCs shift between these two extreme phenotypes. The contractile SMC phenotype is the most abundant in blood vessels of adult organisms. Contractile SMCs are spindle shaped and have muscle characteristics, i.e. contraction in response to mechanical and biochemical stimuli. Synthetic SMCs, with a fibroblast-like appearance, proliferate and produce extracellular matrix components. The phenotypic plasticity is considered to play an important role in angiogenesis, maintenance of blood vessel wall integrity, and the development of certain pathologies.

Although a number of proteins, such as desmin, are expressed in smooth muscle as well as in heart and skeletal muscle tissue, some proteins are specifically expressed in smooth muscle cells only (reviewed in Shanahan and Weissberg 1998). Examples are telokin (Herring and Smith 1996; Smith et al. 1998), α-smooth muscle actin (α-SMA)(Gabbiani et al. 1981), calponin (Takahashi et al. 1991), Crp2/SmLim (Yet et al. 1998), SM22α (Liet et al. 1996 ), smoothelin (Van der Loop et al. 1996) and smooth muscle myosin heavy chain (SM-MHC, Nagai et al. 1989).

Studies towards gene expression in different human tissues have led to the understanding that many proteins are up- or down-regulated in a tissue-specific fashion, i.e. depending on cell type and developmental stage. The feature of tissue-specific induction and repression of gene expression has been employed to target DNA coding sequences to specific cells while the transgene remains silenced in other cells, thus avoiding unwanted side-effects of the expressed transgene product. To date however, a major disadvantage of such highly tissue-specific regulatory elements is the relatively low expression level of the transgene. In addition, targeted gene expression often depends on the physiological/differential or developmental stage of tissues. Subtle differences in cellular features, which are characteristic for such a stage, can strongly influence gene expression levels. Implementation of specific transcriptional control elements that can respond to developmental stages and different phenotypes can diminish the negative effects of such stage-specific features.

Smooth muscle cells and particularly vascular SMCs, are key cells in a number of different diseases affecting the vascular system such as hypertension, atherosclerosis and restenosis after percutaneous transluminal coronary angioplasty (PTCA) (reviewed in Ross 1993; Nikol et al. 1996; Bauters 1995). These disorders, which are widely spread in the western world, result in an impaired blood circulation due to obstruction of the blood vessels (atherosclerosis and restenosis) or due to a decreased output of the heart muscle (hypertension). Transfer to and expression of therapeutic genes in vascular tissues, interfering with the above mentioned pathologies, holds promise for the cure of these diseases.

In general, atherosclerosis starts as an injury of the blood vessel. The endothelial layer is disrupted and a mixture of fatty materials (so-called fatty streak) and macrophages aggregates. This substance, together with the exposure to blood-borne factors, induces a reorganization of the gene expression program of the SMCs, resulting in a phenotypic shift from contractile to proliferative, and finally in migration of these cells. The SMCs of the media move to the luminal side of the fatty streak and start to proliferate and build-up a number of smooth muscle layers that are positioned on top of the fatty streak. This multilayer aggregate is called an atherosclerotic plaque. Although the plaque formation can be seen as a reinforcement of the vascular wall at a spot where the wall has been damaged, the result is a narrowing of the lumen and a reduction of the bloodflow. The reduced blood flow will exercise more shear and thus increase the chances of new injury to the arterial wall.

Restenosis occurs in patients treated with PTCA. The mechanical stretching of the coronary arteries by a balloon, to increase the lumen of these vessels, disrupts the endothelium and induces small tears in the vascular tissue, thus providing access for blood-borne factors to medial SMCs. Similar to atherosclerosis, this results in the migration to and proliferation of SMCs in the lumen of the artery. During restenosis the endothelium layer is usually not distinguishable from SMCs. Eventually, occlusion of the coronary artery can be more severe than the original state.

Hypertension is the result of an increased tonus of the vascular smooth muscle cells resulting in an increased blood pressure. The high blood pressure induces changes in the heart muscle (hypertrophy) and the vascular system (increased chance of atherosclerosis). The cause and mechanism(s) of hypertension are poorly understood and intervention is based on dealing with the effects rather than the cause of the disease.

Since SMCs and in particular vascular smooth muscle cells are involved in a number of diseases they form attractive target cells for the therapeutic intervention of these diseases. In addition, since SMCs, and in particular vascular smooth muscle cells, are closely associated with the circulatory system they also provided an attractive target as a protein factory, excreting therapeutic proteins into the blood stream. To date it has not been possible to target expression of transgenes (nucleic acid of interest) specifically to certain types of smooth muscle cells.

The present invention discloses novel human promoters and the uses thereof in targeting transgene expression to smooth muscle cells that express endogenous smoothelin protein. In the natural context these promoters control the expression of transcripts encoded by two smoothelin open reading frames. The invention further discloses the use of at least one of said promoters in distinguishing subsets of SMCs. In another aspect the invention provides a nucleic acid delivery vehicle for the tissue specific expression of transgenes using a human smoothelin promoter together with nucleic acids encoding therapeutic transgenes.

The invention therefore provides smooth muscle cell specific promoters that can be used to express foreign genetic material specifically in smooth muscle cells. More in particular the invention provides smooth muscle cell specific promoters that can be used to express foreign genetic material specifically in contractile smooth muscle cells. Contractile SMCs comprise the majority of the SMC. Contractile SMCs are non-dividing SMCs. The term "specific expression" of a nucleic acid of interest is meant to indicate that said nucleic acid of interest is capable of being expressed in a certain cell type whereas it is not capable of being expressed in another cell type.

In one aspect the invention provides smooth muscle cell specific promoters that can be used to express foreign genetic material specifically in vascular contractile smooth muscle cells and/or visceral contractile smooth muscle cells. Such foreign genetic information may be introduced into the target cells through the use of a nucleic acid delivery vehicle. Therefore in one embodiment the invention provides a nucleic acid delivery vehicle comprising nucleic acid of interest capable of expressing specifically in a contractile smooth muscle cell. Preferably said nucleic acid of interest is capable of expressing specifically in a vascular contractile smooth muscle cell and/or a visceral contractile smooth muscle cell. Said specificity of expression allows the artisan to tailor the expression of a nucleic acid of interest toward a specific cell. Preferably in a specific part of the body. Said specificity is desired, for instance, in applications where expression of the nucleic acid of interest in other cells or other parts of the body is undesired. Control over expression of a nucleic acid of interest is for instance desired when said nucleic acid encodes nitric oxide synthetase activity. The product, nitric oxide, is an effector molecule in a large number of different processes in the body. In a therapeutic setting one of the desired effects of nitric oxide is sought whereas a number of other effects are not sought. One of the desired effects of nitric oxide sought in a cardiovascular application is for example but not limited to, the reported vasodilatation inducing effect. One of the undesired effects of nitric oxide not sought in a cardiovascular application is for instance but not limited to the apoptosis inducing effect on at least some cells of the immune system. A preferred way to limit the undesired effects is to allow expression of the synthetase mentioned above only in a subset of cells. In the present invention such targeted expression is achieved through allowing expression of the nucleic acid only in contractile smooth muscle cells. In a preferred embodiment the invention provides methods for allowing expression of the nucleic acid of interest essentially only in vascular contractile smooth muscle cells and/or visceral contractile smooth muscle cells. As the artisan will be aware, the capability to allow expression of a nucleic acid of interest only in certain smooth muscle cells is of interest to many other applications than those mentioned above. The present invention makes the development of such applications possible. In one embodiment the invention provides a nucleic acid delivery vehicle delineated above, comprising a smoothelin gene promoter, or a functional part, derivative and/or analogue thereof. In a preferred embodiment of the invention said smoothelin gene is derived from a human. For use in human cells a human smoothelin gene promoter is preferred, however, an equivalent promoter such as a smoothelin gene promoter from a primate, mammal or other higher eukaryote may also be used.

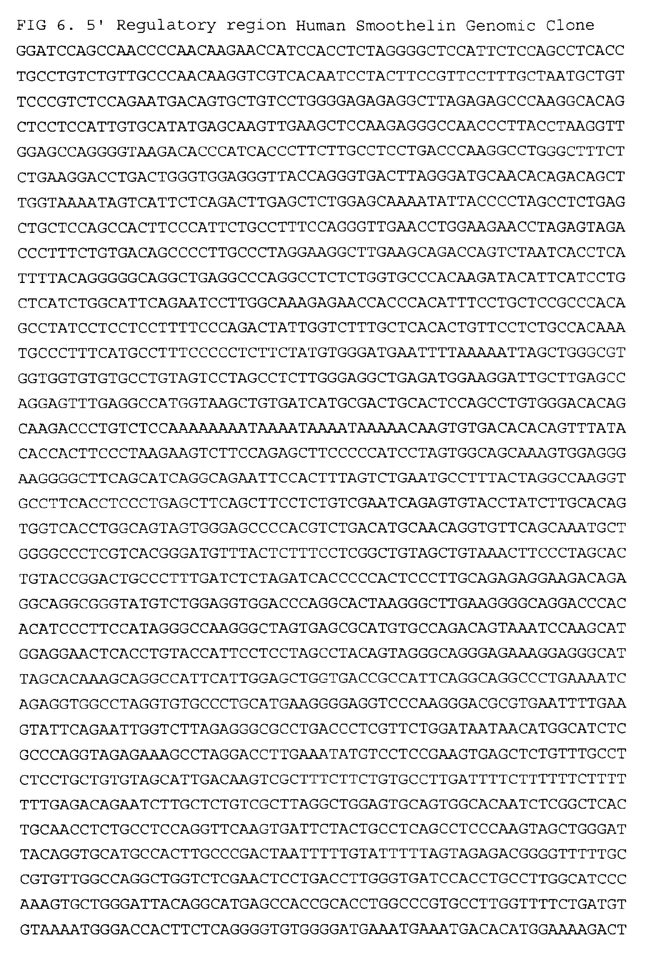

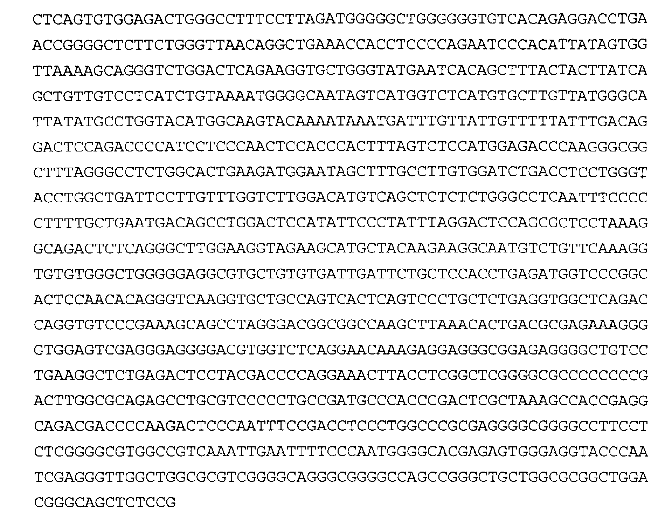

In a preferred embodiment said promoter comprises the promoter driving smoothelin B expression, or a functional part, derivative and/or analogue thereof. Said promoter driving smoothelin B expression being capable of allowing essentially specific expression of a nucleic acid of interest in a contractile smooth muscle cell, preferably a vascular contractile SMC. Preferably, said promoter driving smoothelin B expression comprises a sequence as depicted in figures 6, 7, 8, 9 and/or 10, or a functional part, derivative and/or analogue thereof. Preferably, said promoter driving smoothelin B expression comprises a genomic structure of the human smoothelin gene downstream of the first putative transcription start site, the first two exons of smoothelin-B, the entire first intron and/or a 5' part of the second intron.

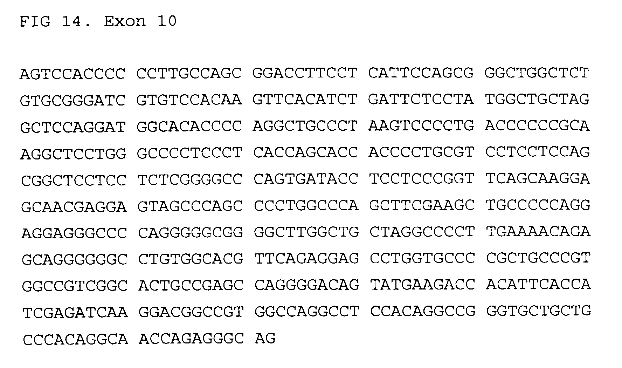

In another embodiment said promoter comprises the promoter driving smoothelin A expression, or a functional part, derivative and/or analogue thereof. Said promoter driving smoothelin A expression being capable of allowing essentially specific expression of a nucleic acid of interest in a contractile smooth muscle cell, preferably a visceral contractile SMC. Preferably said promoter driving smoothelin A expression comprises a sequence as depicted in fig 11, 12, 13, and/or 14, or a functional part, derivative and/or analogue thereof. Preferably, said promoter comprises sequences from intron 9 of the smoothelin B gene.

A functional part of a smoothelin promoter is a part of said promoter capable of conferring essentially the same specificity of expression in kind not necessarily in amount on a nucleic acid of interest. A functional part of a smoothelin promoter is also a part capable of conferring onto another promoter a capability of expression in a vascular and/or visceral contractile smooth muscle cells. Needless to say that a smoothelin promoter or a part thereof can be combined with other promoters or parts thereof to generate novel promoters with essentially the same kind of specificity of expression in kind not necessarily in amount. Combination may also yield promoters having novel specificities, but which share with the smoothelin promoter the capacity to express a nucleic acid of interest in a contractile smooth muscle cell. A suitable derivative of a smoothelin promoter of the invention is a promoter derivable from a smoothelin promoter through nucleic acid substitution, capable of expressing a linked nucleic acid of interest specifically in contractile smooth muscle cells. Suitable analogues of a smoothelin promoter are promoters capable of expressing a linked nucleic acid of interest specifically in contractile smooth muscle cells. Such analogues may be generated synthetically and have incorporated in them nucleic acid sequences capable of binding trans-acting factors capable of binding to a smoothelin promoter. Such trans-acting factor binding sites may be found using techniques known in the art, such as but not limited to footprinting and DNAseI hypersensitivity.
A smoothelin promoter, or a functional part, derivative and/or analogue thereof, can be a part of a locus control region.

A nucleic acid delivery vehicle of the invention preferably comprises a virus-like particle. Although non-viral methods of gene transfer have proven to be very effective in vitro, in vivo results are as yet disappointing. Nucleic acid delivery vehicles comprising virus-like particles are currently seen as the most promising vehicles for in vivo use. In a preferred embodiment said virus-like particle is an adenovirus particle, an adeno-associated virus particle or a retrovirus particle, or a functional part, derivative and/or analogue thereof. A virus particle comprises various functional parts. There are parts for binding to a cell surface. Parts that aid entry of said particle into a cell and parts capable of condensing nucleic acid. A suitable derivative may be generated through neutral amino acid substitution. Suitable analogues are particles comprising a similar part as said virus-like particle.

The present invention is exemplified on the based of adenovirus vectors but is not limited to such vectors. In the examples given *infra* to illustrate the present invention, said recombinant adenoviral vectors are derived from human adenovirus type 5. Typically, one would like to optimise the delivery of the nucleic acid defined *supra,* particularly to cells of the vessel wall, particularly smooth muscle cells. For this reason in one embodiment of the invention the nucleic acid delivery vehicle comprises a tissue tropism determining part, preferably a fiber protein or a part thereof, derived from an adenovirus of a different subgroup than subgroup C, the subgroup that adenovirus serotype 5 belongs to. Preferably, said different subgroup is subgroup B, although subgroup D and/or F are also suitable. Preferably, said adenovirus of subgroup B is adenovirus 16 or adenovirus 35. It is to be understood, however, that those skilled in the art will be able to apply other viral vectors, such as other recombinant adenoviral vectors, without departing from the invention. Methods for the construction of recombinant adenoviral vectors according to the invention and for their propagation on useful packaging cells have been described in patent applications EP 0 707 071 and WO 97/00326, incorporated herein by reference. Other examples of vectors and packaging systems useful in the invention include, but are not limited to, those given in patent applications WO 93/19191, WO 94/28152, WO 96/10642, and WO 97/04119.

In a preferred embodiment a nucleic acid delivery vehicle of the invention comprises an adenovirus particle comprising nucleic acid derived from adenovirus, i.e. an adenovirus vector. In a preferred embodiment the invention provides a method for producing said nucleic acid derived from adenovirus, comprising welding together, preferably through homologous recombination, two nucleic acid molecules comprising partially overlapping sequences wherein said overlapping sequences allow essentially only one homologous recombination which leads to the generation of a physically linked nucleic acid comprising at least two functional adenovirus inverted terminal repeats, a functional encapsulation signal and a nucleic acid of interest or functional parts, derivatives and/or analogues thereof. In a preferred embodiment at least one of said at least two nucleic acid molecules comprises nucleic acid encoding at least a tissue tropism determining part of an adenovirus, preferably a part of a fiber protein of an adenovirus of subgroup B, D or subgroup F.
An important aspect in this embodiment of the invention is that said partially overlapping sequences allow essentially only homologous recombination leading to the generation of a functional adenovirus vector capable of being replicated and packaged into adenovirus particles in the presence of the required transacting functions. With essentially only one is meant that said overlapping sequences in each nucleic acid comprise essentially only one continuous sequence wherein homologous recombination leading to the generation of a functional adenovirus may occur. Within said continuous sequence the actual number of homologous recombination events may be higher than one. Non continuous overlapping sequences are not desired because they reduce the reliability of said method. Non continuous overlapping sequences are also not desired because they reduce the overall efficiency of said method, presumably due to the generation of undesired homologous recombination products.

A preferred embodiment of the invention provides a method for generating an adenovirus vector wherein both of said nucleic acid molecules comprise only one adenovirus inverted terminal repeat or a functional part, derivative and/or analogue thereof. In one aspect one or both of said two nucleic acid molecules have undergone modifications prior to said welding together. Said modification may include the welding together of different nucleic acid molecules leading to the generation of one or both of said two nucleic acid molecules. In a preferred embodiment said different nucleic acids are welded together through homologous recombination of partially overlapping sequences. In a further aspect said welding together is performed in a cell or a functional part, derivative and/or analogue thereof. Preferably said cell is a mammalian cell. More preferably, said welding together is performed in a cell expressing E1-region encoded proteins. Preferably said cell is a PER.C6 cell (ECACC deposit number 96022940) or a derivative thereof. In a preferred embodiment said nucleic acid molecules are not capable of replicating in said mammalian cell prior to said welding together. Said replication is undesired since it reduces the reliability of the methods of the invention presumably through providing additional targets for undesired homologous recombination. Said replication is also not desired because it reduces the efficiency of the methods of the invention presumably because said replication competes for substrate or adenovirus transacting functions with the replication of said adenovirus vector.

In a preferred embodiment, one of said nucleic acid molecules is relatively small and the other is relatively large. This configuration is advantageous because it allows easy manipulation of said relatively small nucleic acid molecule allowing for example the generation of a large number of small nucleic acid molecules comprising different nucleic acid of interest for instance for the generation of an adenovirus vector library. Said configuration is also desired because it allows the production of a large batch of quality tested large nucleic acid molecule. The amplification of large nucleic acid molecules for instance in bacteria is difficult in terms of obtaining sufficient amounts of said large nucleic acid. The amplification of large nucleic acid molecules for instance in bacteria is also difficult to control because a small modification of said large nucleic acid is not easily detected. Moreover, for reasons not quite understood some large vectors are more stable in bacteria or yeasts than others. Said configuration however, allows the generation of a standard batch of a large nucleic acid molecule which can be thoroughly tested, for instance through generating a control adenovirus of which the efficiency and the reliability of production is known, and determining said parameters of a new batch of large nucleic acid molecule. Once validated, said batch may be used for the generation of a large number of different adenovirus vectors through combining said large molecule with a large number of different small nucleic acid molecules. Said system therefore also allows for the selection and/or manipulation of vectors comprising a large nucleic acid molecule of the invention to allow a suitable yield of intact large nucleic acid.

In another embodiment said cell comprising nucleic acid encoding E1-region proteins further comprises a nucleic acid encoding an adenovirus E2-region and/or an adenovirus E4-region protein. Preferably, said cell further comprising nucleic acid encoding an adenovirus E2-region and/or an adenovirus E4-region protein is a derivative of PER.C6. The presence of said nucleic acid encoding an adenovirus early region protein allows the deletion of part of said nucleic acid from said adenovirus vector, thus rendering said adenovirus vector less immunogenic and/or less capable of replicating in a target cell and/or leave more space in the vector for inserting foreign genetic material.

In one aspect of the invention said nucleic acid derived from adenovirus comprises a minimal adenovirus vector or an Ad/AAV chimeric vector. A minimal adenovirus vector comprises at least two adenovirus terminal repeats, an adenovirus packaging signal and optionally a nucleic acid of interest. A chimeric Ad/AAV vector comprises at least two adeno-associated virus inverted terminal repeats, an adenovirus packaging signal and optionally a nucleic acid interest. Ad/AAV chimeric vectors comprise the capacity to persist for a prolonged period of time in cells compared to the classical adenovirus vectors.

In another aspect the invention provides a nucleic acid delivery vehicle defined *supra*, further comprising a nucleic acid of interest. Preferably said nucleic acid of interest encodes an apolipoprotein, a nitric oxide synthetase, a ceNOS, a herpes simplex virus thymidine kinase, an interleukin-3, an interleukin-1α, an (anti)angiogenesis protein such as angiostatin, an anti-proliferation protein such as but not limited to GATA6, a vascular endothelial growth factor (VGEF), a basic fibroblast growth factor (bFGF), a hypoxia inducible factor 1α (HIF-1α), a PAI-1 or a smooth muscle cell anti-migration protein.

In another aspect the invention provides a cell provided with nucleic acid of interest capable of expressing specifically in a contractile smooth muscle cell. Preferably said cell is a contractile smooth muscle cell. Preferably a vascular and/or a visceral contractile smooth muscle cell. The invention further provides a cell comprising a nucleic acid delivery vehicle according to the invention. In one embodiment said cell is capable of producing said nucleic acid delivery vehicle. Said cell may be an adenovirus packaging cell. Preferably said adenovirus packaging cell comprises a nucleic acid encoding an adenovirus early region 1 encoded protein, or a functional part, derivative and/or analogue thereof. Said adenovirus packaging cell may further comprise nucleic acid encoding an adenovirus early region 2 and/or an adenovirus early region 4 encoded protein, or a functional part, derivative and/or analogue thereof. A "functional part" a "functional derivative" and a "functional analogue" of an adenovirus early region encoded protein means a protein capable of performing the same function as said adenovirus early region encoded protein in kind not necessarily in amount. Preferably said adenovirus packaging cell is a PER.C6 cell (ECCAC deposit number 96022940) or a derivative thereof.

The invention further provides a method for providing a cell with a nucleic acid of interest capable of expressing specifically in a contractile smooth muscle cell comprising contacting said cell or a precursor thereof with a nucleic acid delivery vehicle according to the invention.

The invention also provides the use of a nucleic acid delivery vehicle according to the invention, for the preparation of a pharmaceutical. Provided is further the use of a nucleic acid delivery vehicle of the invention for the preparation of a pharmaceutical for the treatment of a cardiovascular disease.

The invention further provides a promoter of a smoothelin gene, or a functional part, derivative and/or analogue thereof. Preferably said promoter comprises a sequence as depicted in figures 6, 7, 8, 9, 10, 11, 12, 13 and/or 14, or a functional part, derivative and/or analogue thereof.

In another aspect the invention provides the use of a promoter, or a functional part, derivative and/or analogue thereof of a smoothelin gene, for providing a nucleic acid of interest with the capacity to express specifically in a contractile smooth muscle cell.

In yet another aspect the invention provides a nucleic acid as depicted in figures 6, 7, 8, 9, 10, 11, 12, 13 and/or 14.

The use of smooth muscle-specific promoter elements, as can be derived from the smoothelin promoter, will aid in the development of treatment modalities of the disorders mentioned above. In addition, such a promoter may be a candidate for intervention in other diseases that are the consequence of changes in smooth muscle cell phenotype.

In one aspect the invention provides the use of a sequence capable of regulating smoothelin expression for obtaining a smoothelin-like expression pattern of a linked nucleic acid of interest. Two isoforms of smoothelin are expressed in a tissue-specific manner: a 59 kDa isoform, further referred to as smoothelin-A, in visceral and urogenital tissues, and a 110 kDa isoform, referred to as smoothelin-B, in vascular tissues. The two isoforms might be the result of 1) two genes coding for proteins that share at least the domain reacting with the anti-smoothelin antibody, 2) differences in posttranscriptional processing, 2) alternative splicing, 3) a dual promoter system. Radiation hybrid mapping and fluorescence in situ hybridisation (FISH) with the smoothelin-A cDNA as well as a genomic clone selected from a genomic human cosmid library, demonstrated that smoothelin is coded for by a single copy gene located on chromosome 22q12.3 (Engelen et al., 1997).

Transfer of genes into mammalian cells can be achieved by a number of technologies such as using carrier-like gold particles, or lipo-carriers, or by viral particles such as retroviruses and adenoviruses. The adenoviral DNA has been modified to create suitable gene transfer vehicles, and at present adenoviral vectors are available that efficiently transfer genes into a variety of cells. However, the current generation of adenoviral vectors has two major drawbacks: 1) After systemic delivery in mammals, adenoviral particles are sequestered by the liver; 2) Adenoviral particles cause immuno-toxic effects after (repeated) injection. In cases where expression in hepatic cells is not desired, the use of a tissue-specific promoter becomes mandatory. At present, considerable research effort focuses on modification of the adenoviral capsid in such a way that other cell types can be targeted.
The immune response can be directly towards the injected particles but also against the capsid proteins of the adenovirus, like hexon, penton and fiber that are expressed late in the viral production cycle. These late expressed proteins are known to be involved in the immunogenicity of the virus. When the adenoviral particles are applied by a number of injections over a period of time, a booster effect may occur. The vast majority of individuals have had previous exposure to adenoviruses, especially the well investigated adenovirus serotypes 5 and type 2 (Ad5 and Ad2) or immunologically related serotypes. Importantly, these two serotypes are also the most extensively studied for use in human gene therapy. The usefulness of these adenoviruses or cross-immunising adenoviruses to prepare gene delivery vehicles may be seriously hampered, since the individual to which the gene delivery vehicle is provided, will raise a neutralising response to such a vehicle before long. There is thus a need in the field of gene therapy to provide gene delivery vehicles, preferably based on adenoviruses, which do not encounter pre-existing immunity and/or which are capable of avoiding or diminishing neutralising antibody responses. Thus the invention provides a nucleic acid delivery vehicle comprising at least one of the adenovirus serotype 35 elements or a functional equivalent thereof, responsible for avoiding or diminishing neutralising activity against adenoviral elements by the host to which the gene is to be delivered and a gene of interest. A functional equivalent/homologue of adenovirus 35 (element) for the purpose of the present invention is an adenovirus (element) which, like adenovirus 35, encounters pre-existing immunity in less than about 10% of the hosts to which it is administered for the first time, or which is capable in more than about 90% of the hosts to which it is administered to avoid or diminish the immune response. Typical examples of such adenoviruses are adenovirus serotypes 34, 26 and 48. A gene delivery vehicle may be based on adenovirus 35 or a functional homologue thereof, but it may also be based on another backbone, such as that of adenovirus 2 or 5, as long as it comprises at least one of the elements from adenovirus 35 or a functional equivalent thereof, which leads to the diminishing of the immune response against such an adenovirus 2 or adenovirus 5 based gene delivery vehicle. Of course the gene delivery vehicle may also comprise elements from other (adeno) viruses, as long as one replaces an element which could lead to immunity against such a gene delivery vehicle by an element of adenovirus 35 or a functional homologue thereof, which has less of such a drawback and which preferably avoids such a drawback. In the present invention a gene delivery vehicle is any vehicle that is capable of delivering a nucleic acid of interest to a host cell. It must, according to this aspect of the invention comprise an element of adenovirus 35 or a functional equivalent of such an element, which must have a beneficial effect regarding the immune response against such a vehicle. Basically all other elements making up the vehicle can be any elements known in the art or developed in the art, as long as together they are capable of delivering said nucleic acid of interest. In principle the person skilled in the art can use and/or produce any adenoviral products or production systems that can or have been applied in the adenoviral field. In addition, an immune response can be elicited against the recombinant transgene products that are expressed from the adenoviral genome. Transgenes that are under the control of a non-tissue-specific promoter, like the immediate early promoter of the Cytomegalovirus (CMV), are expressed in a great variety of tissues. This could pose a problem to the organism in those cases where the transgene encodes a toxic protein. Therefore, it offers considerable advantages to have tissue-specific control elements that limit the expression of a particular transgene to those tissues or areas to be targeted.

In SMCs a limited number of genes have been found to be transcriptionally upregulated (reviewed in Shanahan and Weissberg 1998). Examples are telokin (Herring and Smith 1996; Smith et al. 1998), desmin, α-SMA, calponin, Crp2/SmLim (Yet et al. 1998), SM22α, smoothelin (Van der Loop et al. 1996) and SM-MHC. In contrast to vascular endothelial cells, not much is known about the regulation of genes that are expressed exclusively in SMCs. In fact, only a few promoters have been cloned, studied and applied in experiments for SMC specific expression of recombinant transgenes. Vascular SMC-specific control elements have been tested in mammalian cells and in transgenic animal studies. This work has been done with promoter(elements) of the smooth muscle myosin heavy chain, the α-SMA and the SM22α genes (Li et al 1996b; Madsen et al., 1998; Mack et al.,1999). The α-SMA promoter was cloned from human, rat, mouse and (Blank et al. 1992; Min et al. 1990; Reddy et al. 1990; Carrol et al. 1986). Another promoter that has been applied in many studies is the SM22α promoter (Yamamura et al. 1997; Kemp et al. 1995). The specificity of expression of SM22α is relatively low, since it is expressed in most SMCs phenotypes and (at a low level) in heart. The SM22α promoter has been used in transgenic mice driving a LacZ reporter construct (Li et al. 1996a) and in transcriptional targeting of SMCs in vivo with replication defective adenovirus (Kim et al. 1997), also transactivating the expression of LacZ. Other examples are the SM-MHC promoter and the telokin promoter. Promoters appear to be rather complex with important elements in the first intron of the gene. Expression of reporter genes was found in subpopulations of the SMCs (Madsen et al., 1998), or only during particular developmental stages (Li et al., 1996a).

These promoter studies demonstrate that gene regulation in SMCs reflects the phenotypic variety of these cells. In addition, Mano and coworkers (1999) recently described a transcription factor, GATA6, that appears to be capable of inhibiting SMC proliferation after balloon induced injury in rat aorta. The invention therefore provides a nucleic acid delivery vehicle of the invention further comprising a nucleic acid encoding GATA6 or a functional part, derivative and/or analogue thereof. Said nucleic acid delivery vehicle being capable of at least in part essentially arresting contractile SMCs in the contractile state. Said nucleic acid delivery vehicle at least in part being capable of preventing restenosis upon delivery of said nucleic acid delivery vehicle comprising a nucleic acid encoding GATA6 to SMCs of the vessel wall treated with PTCA.

Two isoforms of smoothelin have been identified so far: smoothelin-B, 110 kDa, expressed only in vascular SMCs, and smoothelin-A, a 59 kDa protein, found in visceral SMCs (Van der Loop et al., 1996; Van Eys et al., 1997; Wherens et al., 1997). The expression pattern of smoothelin is similar to that of SM22α and calponin. Both proteins co-localise, like smoothelin, with α-SMA indicating an interaction with this molecule. During embryogenesis these proteins are transiently expressed in heart and somites, probably in a period that the cells of these organs have only primitive muscular characteristics. In adults, smoothelin is solely present in fully differentiated contractile SMCs (Van der Loop et al., 1996; Van Eys et al., 1997). No expression of smoothelin was detected in SMC-like, such as myofibroblasts and myoepithelial cells, nor in adult skeletal and cardiac muscle. The relation/association between smoothelin and other SMC-specific proteins such as SM22α, calponin and α-SMA is not clear. The cDNAs of both isoforms have been cloned and characterised. The two isoforms are transcribed from a single copy gene, which is located on chromosome 22q12.3 (Engelen et al. 1997). Two isoforms of human smoothelin can be distinguished that are each under the control of their own respective promoter all located within one single genomic region.

In one aspect of the invention, a gene encoding Nitric Oxide Synthase (NOS) may be expressed in SMCs or endothelial cells leading to the release of biologically active NO. The art teaches that NO may act as a signal in the angiogenic response of endothelial cells to growth factors like bFGF and VEGF. A decreased NO synthesis in endothelial cells may limit new blood vessel formation in patients with endothelial dysfunction. It is therefore an object of the present invention to provide means for local expression of NOS in the arterial wall.

In another aspect of the invention, a gene encoding a so-called prodrug- activating enzyme can be expressed specifically in SMCs providing the conversion of a harmless prodrug to a toxic and growth inhibitory drug that prevents the outgrowth of proliferative cells. The prodrug-activating enzyme is secreted from the producing cells and exerts its effect at some distance from the cells expressing the enzyme. Cells that are prone to growth are hereby affected while non-proliferative cells are not.

The art does not provide a means for transcriptional targeting of transgenes to terminally differentiated SMCs. If promoters from telokin, SM-MHC or SM22 are used different subsets of SMCs other and even perhaps all SMCs will be found to express the particular transgene and the use of these promoters will not distinguish between the different known subsets. Therefore the art does also not provide a suitable marker for SMC subtyping.

Until the present invention there are no promoters available that have been found suitable to produce recombinant proteins specifically in SMCs. It is therefore clearly desirable to have such methods for specific targeting of SMCs that are differentiated to their final stage, while cells that are proliferating are not affected by the particular transgene.

Thus the invention provides a method for producing recombinant proteins in differentiated contractile SMCs, while cells that proliferate will not express genes that are transcriptionally controlled by said promoter. In another aspect of the invention, the invention provides a method for producing a recombinant protein in a human smooth muscle cell, comprising providing a human cell with a chimeric gene driven by a promoter fragment from the smoothelin gene, or a derivative thereof.

The invention further provides a use according to the invention, wherein said promoter comprises sequences upstream of the open reading frame of the gene encoding both isoforms of human smoothelin, whereas the smoothelin-B open reading frame is situated upstream of the open reading frame encoding smoothelin-A.

The invention further provides a use according to the invention, wherein said promoter comprises sequences of the genomic structure of the human smoothelin gene downstream of the first putative transcription start site, including the first two exons of smoothelin-B and the entire first intron and a 5' part of the second intron.

### EXAMPLES

To illustrate the invention, the following examples are provided, not intended to limit the scope of the invention.

### Example 1. Identification of smoothelin-B

### Tissue samples and cell culture.

Normal adult human tissues, obtained at autopsy, and tissues from a variety of animals (pig, dog, cattle, rabbit, rat, mouse) were immediately frozen and stored at -80°C until use. Human SMCs were obtained from vena, iliac, uterine, and mammary artery by enzymatic dispersion (collagenase/pancreatin: Life Technologies, Gaithersburg, MD, USA). Cells were cultured in Dulbecco's Modified Essential Medium supplemented with 15% foetal calf serum (Life Technologies).

For immunohistochemistry tissues were mounted in Tissue-Tek (OCT- compound; Miles Inc. Elkhart, IN, USA), and 3 to 5 mm thick sections were cut at -25°C and air-dried overnight at room temperature or immediately fixed with methanol (at-20°C for 5 min) followed by acetone (-20°C for 30 sec) and air-dried for 3 hours before use. Cultured cells were grown on cover slips and fixed in methanol/acetone as described above.

### Antibodies

Antibodies used in this study were:
1. The mouse monoclonal antibody R4A directed against smoothelin (Van der Loop et al., 1996).
2. The mouse monoclonal antibody C6G directed against human smoothelin. To generate the antibody mice were immunised with recombinant human smoothelin. Recombinant smoothelin was produced as described previously (Van der Loop et al., 1996). Fusion procedure and cloning of the hybridomas were performed according to standard protocols (Köhler and Milstein, 1975). The monoclonal antibody C6G was selected on basis of its specific reactivity pattern with a selection of human cardiac, skeletal and smooth muscle tissues. C6G is an antibody of the IgG1-subclass (Mouse Mab Isotyping kit; Life Technologies).
3. Polyclonal rabbit antiserum (pDes) to chicken gizzard desmin (Ramaekers et al., 1985).
4. Monoclonal antibody sm-1 to smooth muscle actin was purchased from Sigma Immuno Chemicals (St. Louis, MO, USA) (Skalli et al., 1986).
In addition, rhodamine-labeled phalloidin (purchased from Molecular Probes Inc. Eugene, OR, USA) was used to stain actin stress-fibers.

### Immunohistochemistry

Immunofluorescence as well as immunoperoxidase staining has been performed on tissues of different species. In all cases 3 to 5 mm thick cryostat sections were used. The sections were pretreated for 5 min with 0.5% Triton X-100 (BDH Chemicals Ltd., Poole, UK) in phosphate buffered saline (PBS: 137 mM NaCl, 13 mM Na₂HPO₄.2H₂O, 3 mM KH₂PO₄ pH 7.4; Merck, Darmstadt, Germany), followed by a PBS washing step for 5 min. Methanol/acetone fixed sections were used without Triton X-100-pretreatment.

Tissue sections or fixed cells were incubated with the primary antibody for 30 min at room temperature, washed with PBS and incubated with a secondary antibody conjugated to either fluorescein isothiocyanate (FITC) (goat anti-mouse-IgG-FITC, Southern Biotechnology Associates Inc., Birmingham, AL, USA) and Texas Red (TR) (goat anti-rabbit-Ig-TR/goat anti-mouse-IgG1-TR; SBA). The secondary antibodies were applied for 30 min at room temperature. After three washing steps with PBS, the fluorescently stained tissues were mounted in Mowiol (Hoechst, Frankfurt, FRG) or in Kaiser's glycerin-gelatin (Merck) for AEC stained sections.

### Protein gel electrophoresis and Western blotting.

Cultured cells (approximately 10⁶) or about 40 cryostat sections (each 20 mm thick) of fresh frozen tissues were collected, washed with 1 ml PBS and centrifuged for 5 min at 12.000x*g*. After centrifugation the pellet was subjected to a Triton X-100 extraction step. Cells were suspended in 1% Triton X-100, 5 mM ethylenediaminotetraacetic acid disodium salt dihydrate (EDTA; Merck), 0.4 mM phenylmethylsulfonyl - fluoride (PMSF; Merck) in PBS, pH 7.4 and extracted for 5 min on ice. After centrifugation for 5 min at 12.000x*g*, the pellet was washed in 1 ml PBS. After a final centrifugation step (5 min, 12.000x*g*), the cytoskeletal preparation was dissolved by boiling for 4 min in sample buffer (Laemmli, 1970), containing 2.3% sodium dodecylsulfate (SDS) and 5% β-mercaptoethanol (Bio-Rad Laboratories, Richmond, CA, USA). For one-dimensional SDS-polyacrylamide gel electrophoresis (SDS-PAGE) using a Mini Protean II Electrophoresis Cell (Bio-Rad Laboratories) 7.5-10% polyacrylamide slab gels containing 0.1% SDS (Laemmli, 1970) were used. After electrophoretic separation, the proteins were stained with Page Blue 83 (BDH Chemicals Ltd.) or subjected to Western blotting. Culture supernatant of monoclonal antibody R4A or C6G, directed against smoothelin, was used in a 1:5 dilution and immunodetection with horse-radish peroxidase conjugated rabbit anti-mouse-Ig (DAKO A/S) was performed according to standard procedures.

Immunocytochemical studies showed an equally strong reaction of anti-smoothelin monoclonal antibodies R4A and C6g with visceral and vascular smooth muscle tissues (Van der Loop et al. 1996; Van Eys et al. 1997). Western blots of visceral smooth muscle tissues displayed a 59kD protein after incubation with R4A. Breakdown products with a molecular weight of about 40kD were occasionally observed. Western blot analysis of vascular smooth muscle tissues displayed a strong reaction of R4A with a 110kD polypeptide Van Eys et al.1999).

Northern blot analysis of cultured vascular SMCs showed a transcript of approximately 3000 nucleotides hybridising with the visceral smoothelin cDNA probe, whereas a major transcript from visceral SMCs was only approximately 1500 nucleotides ( Van Eys et al, 1999). The appearance of the larger vascular transcripts suggests another smoothelin form which might react with R4A and which is likely the 110kD protein detected in western blots.

### Example 2. Cloning of the human cDNA encoding the vascular smoothelin isoform, smoothelin-B

RNA was extracted from human iliac artery smooth muscle by LiCl extraction (Auffray and Rougeon 1980). Concentration and quality of RNA was evaluated by routine Northern blot analysis using a ³²-P labelled smoothelin cDNA probe. Using primers based on the sequence of the human smoothelin-A cDNA the possibility of alternative splicing was investigated by reverse transcription-PCR (RT-PCR). Five ug of total vascular RNA was subjected to reverse transcription with a set of primers that covered the smoothelin-A cDNA. After heating the RT mixture to 70°C for 5 minutes, 5 ul was used for PCR. Length of the PCR-generated fragments was compared with those generated by PCR of the smoothelin-A cDNA. Rapid amplification of cDNA ends (5' RACE) was applied to investigate the possibility of a 5' extension of the mRNA. Using primers based on sequences of the 5' part of the smoothelin-A cDNA in a 5' RACE system (Life Technologies Inc.). PCR fragments were generated and cloned after SalI/BamHI digestion. Colonies were screened with a genomic clone containing the sequence from -200 to +60 of the smoothelin-A cDNA. Clones containing cDNA inserts were mapped by restriction digestion and sequenced. Sequences were checked by analysis of RT-PCR derived sequence fragments.

Sequence comparison and structural analysis of the putative protein were performed using BLAST, BLAST-X algorithms (Altschul et al. 1990) and protein structure programs PHD and SOPMA (Rost and Sander 1993; Rost and Sander 1994; Geourjon and Deleage 1994; Geourjon and Deleage 1995).

### Northern blot analysis.

Total RNAs of vascular tissues (human, dog, pig) and from primary cell cultures of human arteria iliaca were extracted by LiCl (Auffray and Rougeon, 1980). 10 mg of total RNA was separated on a 2% agarose formaldehyde denaturing gel (Sambrook et al., 1989). RNA was transferred to nitrocellulose (S&S, Basel, Switzerland) and hybridized to the smoothelin-cDNA probe according to standard procedures (Church and Gilbert, 1984). Filters were washed in decreasing SSC (NaCl, Na-citrate) concentrations with a final concentration of 0.1x SSC/0.1% SDS. Probes were ³²P labelled by random priming using a kit (Life Technologies) according to Feinberg and Vogelstein (1983).

### Transfection of smoothelin -BcDNA into COS7 and CHO cells.

The full size smoothelin cDNA was recloned in a pcDNA3 eukaryotic expression vector (Invitrogen, San Diego, USA) [this construct is futher referred to as pSMO888]. Isolated plasmid was purified by CsCl gradient centrifugation (Sambrook et al., 1989). Transfection of exponentially growing CHO and COS7 cells was done with 5 mg purified DNA per 10⁶ cells, using the DOTAP lipocarrier system as suggested by the manifacturer (Boehringer Mannheim, Germany). About 16 and 40 h after transfection cells were fixed with methanol/acetone or harvested after trypsinization. Fixed cells were incubated with rhodamine labelled phalloidine and antibodies directed against smoothelin, vimentin, and desmin as described above. Cells harvested after trypsinisation were collected by centrifugation and prepared for Western blotting analysis as described above.

### Production of smoothelin protein in E.coli and generation of vascular-specific monoclonal antibody.

The smoothelin cDNA (1593 bp clone) was excised from Bluescript II and inserted into the prokaryote expression vectors pQE9/10/11 (Qiagen, Kassel, Germany). Insertion in these vectors results in a fusion of the cDNA encoded protein to a 6-His peptide, allowing a single-step purification of the protein by binding to Ni-agarose. Protein was synthesized during overnight induction with 1 mM IPTG (Life Technologies). Proteins produced in E.coli were obtained by guanidine/urea extraction and purified according to the protocol of the manufacturer (Qiagen). After separation by SDS-PAGE proteins were blotted onto nitrocellulose and identified by antibody R4A as described above.

### Example 3. Cloning and analysis of the genomic structure of the human smoothelin gene

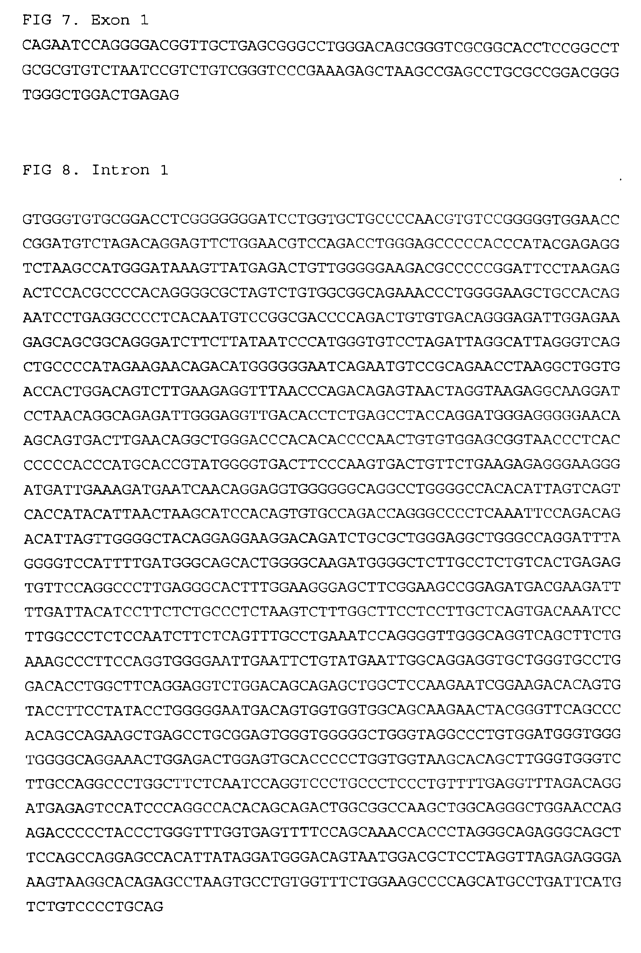

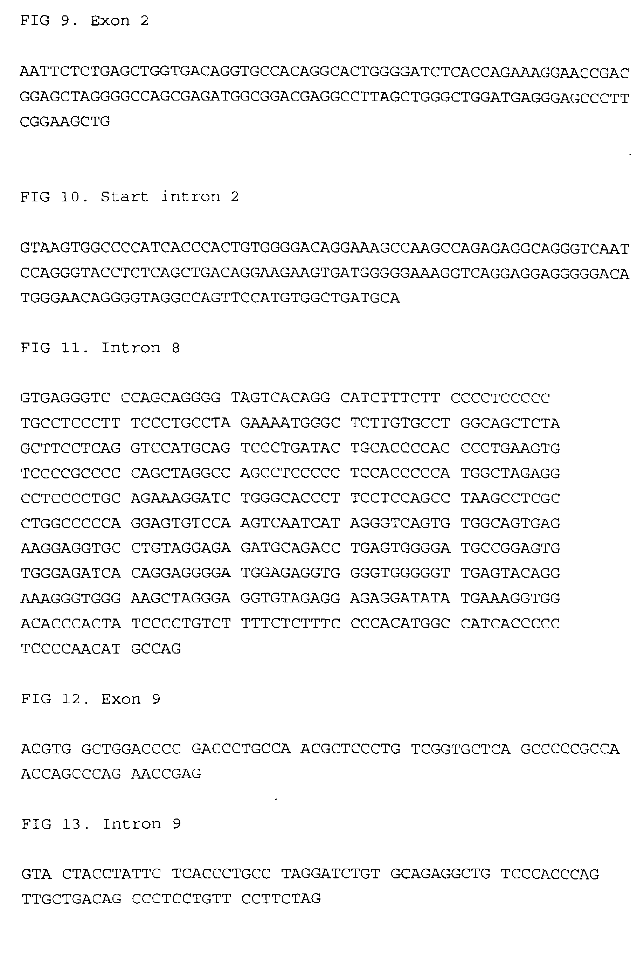

Approximately 10⁵ clones of a human placental cosmid genomic library (Stratagene, La Jolla, USA) were screened with a ³²P labelled smoothelin-A probe. Two positive clones were purified to homogeneity. Restriction analysis showed that the two clones contained an overlap of approximately 30 kb. One clone contained the complete coding region of the smoothelin gene. In addition, an approximate 6 kb 5' flanking sequence was present. This clone was subcloned into pUC19 and exon-containing subclones were identified by colony hybridisation with cDNA fragments. A complete map of the cosmid clone was constructed and exons and their flanking sequences were sequenced using an Applied Biosystems AmpliTaq sequencing kit or using a conventional T7 sequencing kit with 32-P labelled probes. The sequence present upstream of the transcription start site of smoothelin-B is depicted in fig 6. The sequences of exon 1, intron 1 and exon 2 are shown in figs 7, 8 and 9 respectively. A partial sequence of the 2^{nd} intron is shown in fig 10.

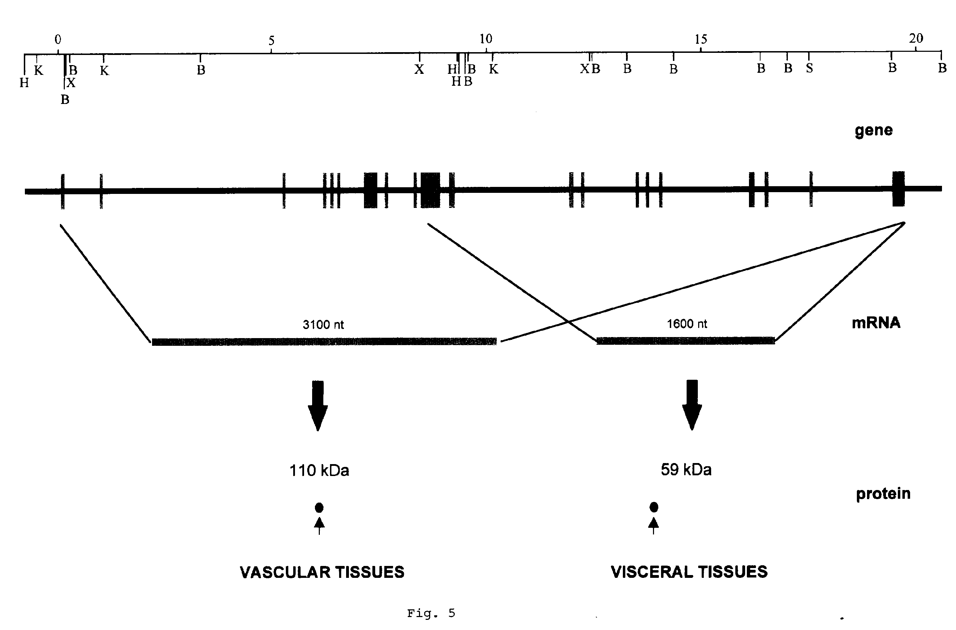

A human cosmid library was screened with a smoothelin-A cDNA probe. Two clones were selected. Endonuclease restriction analysis indicated that the two clones shared about 30 kb. The cosmid was hybridized with a 200 bp probe of the 3' end of the smoothelin-A cDNA and with a 300 bp probe containing the 5' end of the smoothelin-B cDNA. The positive reaction with both probes indicated that the whole smoothelin gene was present in the cosmid clone. A restriction map of the largest clone was constructed (Figure 5) and the DNA was subcloned in pUC19 after BamHI and XbaI digestion. The subclones were tested for the presence of exons by hybridisation with cDNA fragments. Positive subclones were further processed and sequenced (Figure 5). Twenty exons have been positioned (Figure 5). The first two rather small exons are separated from the other exons by a 5 kb intron. Exon 10 is the largest one comprising 526 bp. In the middle of this exon is the start point for the transcription of smoothelin-A (Figure 5). Exons 7 and 20 are also rather large, around 300 bp. All other exons are around 150 bp or smaller. Most exon-intron bounderies have the AGGT recognition sequence (table 1). Between exons 16 and 17 a 50 bp TAAA repeat has been found. The vascular specific part of the smoothelin-B cDNA sequence is coded for by the first nine exons and 5' half of exon 10. The 3'half of exon 10 and the remaining ten exons are shared by both smoothelin isoforms.

The smoothelins are encoded by one single-copy gene. Since it was not clear whether the two transcripts originate by alternative splicing or are induced via a dual promoter system, sequences directly upstream of the transcription initiation sites have been screened for promoter/enhancer elements. The cosmid contains 6 kb upstream of the transcription initiation site of smoothelin-B. No TATA-box has been found 5' of the putative transcription initiation sites of smoothelin-A as well as smoothelin-B. However, a number of promoter/enhancer elements have been identified in both putative promoter sites. Amongst them are CarG(like), AP-2, SP-1, and GATA boxes.

Example 4. Cloning of the BamHI/BamHI regulatory fragment of the human Smoothelin gene in pUC19 (Plasmid #622).

The region upstream of the transcription start of the smoothelin-B mRNA was sequenced. A restriction map waqs constructed and two BamHI sites were used to subclone an approximately 3.5 kb fragment from the smootheline gene containing cosmid into the BamHI site of pUC19. The Cosmid was digested with BamHI and fragments were seperated by agarose gel electrophoresis. Fragments were obtained by isolation from the gel by electrolution and subsequently purified by phenol/chloroform extraction and ethanol precipitation. The vector pUC19 was digested by BamHI, purified and dephosphorylated with calf intestinal phosphatase. The vector and fragment were mixed and ligated by T4 ligase to yield plasmid #622. The BamHI fragment contains exon 1 and de putative promoter of the smoothelin gene.

Example 5. Cloning of the HincII/BamHI regulatory fragment of the human Smoothelin gene in pUC19 (Plasmid #713).

A extensive restriction map of plasmid #622 with HincII would provide a clone containing approximately 1.0 kb of the smoothelin gene upstream of the transcription start site of the smoothelin-B mRNA.

Plasmid #622 was digested with HincII. Fragments were seperated by agarose gel electrophoresis. Fragments were obtained by isolation from the gel by electrolution and subsequently purified by phenol/chloroform extraction and ethanol precipitation. The fragment containing vector and the remaining 1.0 kb insert was religated by T4 ligase, providing a plasmid designat plasmid #713 containing exon 1 and app. 1.0 kb of the putative smoothelin promoter.

### Example 6. Construction of pAdSMO.LacZ.sal,

pAdSMO.ceNOS.pac, pAdSMO.rIL3.pac and pAdSMO.Luc.sal plasmids.
The plasmid pMLPI.TK (described in WO 97/00326) is an example of an adapter plasmid designed for use in combination with improved packaging cell lines like PER.C6 (described in WO 97/00326 and US 08/892,873). First, a PCR fragment was generated from pZipΔMo+PyF101(N⁻) template DNA (described in PCT/NL96/00195) with the following primers: LTR-1 (5'-CTG TAC GTA CCA GTG CAC TGG CCT AGG CAT GGA AAA ATA CAT AAC TG-3') and LTR-2 (5'-GCG GAT CCT TCG AAC CAT GGT AAG CTT GGT ACC GCT AGC GTT AAC CGG GCG ACT CAG TCA ATC G-3'). The PCR product was then digested with BamHI and ligated into pMLP10 (Levrero et al., 1991), that was digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Next, the coding region of the murine HSA gene was inserted. pLTR10 was digested with BstBI followed by Klenow treatment and digestion with NcoI. The HSA gene was obtained by PCR amplification on pUC18-HSA (Kay et al., 1990) using the following primers: HSA1 (5'-GCG CCA CCA TGG GCA GAG CGA TGG TGG C-3') and HSA2 (5'-GTT AGA TCT AAG CTT GTC GAC ATC GAT CTA CTA ACA GTA GAG ATG TAG AA-3'). The 269 bp PCR fragment was subcloned in a shuttle vector using NcoI and BglII sites. Sequencing confirmed incorporation of the correct coding sequence of the HSA gene, but with an extra TAG insertion directly following the TAG stop codon. The coding region of the HSA gene, including the TAG duplication was then excised as a NcoI/SalI fragment and cloned into a 3.5 kb NcoI/BstBI cut pLTR10, resulting in pLTR-HSA10. This plasmid was digested with EcoRI and BamHI after which the fragment, containing the left ITR, the packaging signal, the L420 promoter and the HSA gene, was inserted into vector pMLPI.TK digested with the same enzymes and thereby replacing the promoter and gene sequences, resulting in the new adapter plasmid pAd5/L420-HSA.

The pAd5/L420-HSA plasmid was digested with AvrII and BglII followed by treatment with Klenow and ligated to a blunt 1570 bp fragment from pcDNA1/amp (Invitrogen) obtained by digestion with HhaI and AvrII followed by treatment with T4 DNA polymerase. This adapter plasmid was named pAd5/CLIP.

To enable removal of vector sequences from the left ITR, pAd5/L420-HSA was partially digested with EcoRI and the linear fragment was isolated. An oligo of the sequence 5' TTA AGT CGA C-3' was annealed to itself resulting in a linker with a SalI site and EcoRI overhang. The linker was ligated to the partially digested pAd5/L420-HSA vector and clones were selected that had the linker inserted in the EcoRI site 23 bp upstream of the left adenovirus ITR in pAd5/L420-HSA resulting in pAd5/L420-HSA.sal.

To enable removal of vector sequences from the left ITR, pAd5/CLIP was also partially digested with EcoRI and the linear fragment was isolated. The EcoRI linker 5' TTA AGT CGA C-3' was ligated to the partially digested pAd5/CLIP vector and clones were selected that had the linker inserted in the EcoRI site 23 bp upstream of the left adenovirus ITR resulting in pAd5/CLIP.sal. The vector pAd5/L420-HSA was also modified to create a PacI site upstream of the left ITR. Hereto pAd5/L420-HSA was digested with EcoRI and ligated to a PacI linker (5'-AAT TGT CTT AAT TAA CCG CTT AA-3'). The ligation mixture was digested with PacI and religated after isolation of the linear DNA from agarose gel to remove concatamerised linkers. This resulted in adapter plasmid pAd5/L420-HSA.pac.

This plasmid was digested with AvrII and BglII. The vector fragment was ligated to a linker oligonucleotide digested with the same restriction enzymes. The linker was made by annealing oligos of the following sequence: PLL-1 (5'- GCC ATC CCT AGG AAG CTT GGT ACC GGT GAA TTC GCT AGC GTT AAC GGA TCC TCT AGA CGA GAT CTG G-3') and PLL-2 (5'- CCA GAT CTC GTC TAG AGG ATC CGT TAA CGC TAG CGA ATT CAC CGG TAC CAA GCT TCC TAG GGA TGG C-3'). The annealed linkers was seperately ligated to the AvrII/BglII digested pAd5/L420-HSA.pac fragment, resulting in pAdMire.pac. Subsequently, a 0.7 kb ScaI/BsrGI fragment from pAd5/CLIP.sal containing the sal linker, was cloned into the ScaI/BsrGI sites of the pAdMire.pac plasmid after removal of the fragment containing the pac linker. This resulting plasmid was named pAdMire.sal.

pAd5/L420-HSA.pac was digested with AvrII and 5' protruding ends were filled in using Klenow enzyme. A second digestion with HindIII resulted in removal of the L420 promoter sequences. The vector fragment was isolated and ligated separately to a PCR fragment containing the CMV promoter sequence. This PCR fragment was obtained after amplification of CMV sequences from pCMVLacI (Stratagene) with the following primers: CMVplus (5'-GAT CGG TAC CAC TGC AGT GGT CAA TAT TGG CCA TTA GCC-3') and CMVminA (5'-GAT CAA GCT TCC AAT GCA CCG TTC CCG GC-3'). The PCR fragment was first digested with PstI after which the 3'-protruding ends were removed by treatment with T4 DNA polymerase. Then the DNA was digested with HindIII and ligated into the AvrII/HindIII digested pAd5/L420-HSA.pac vector. The resulting plasmid was named pAd5/CMV-HSA.pac. This plasmid was then digested with HindIII and BamHI and the vector fragment was isolated and ligated to the HindIII/BglII polylinker sequence obtained after digestion of pAdMire.pac. The resulting plasmid was named pAdApt.pac and contains nucleotides -735 to +95 of the human CMV promoter/enhancer (Boshart et al., 1985). Subsequently, the 0.7 kb ScaI/BsrGI fragment from pAd5/CLIP.sal containing the sal linker, was cloned into the ScaI/BsrGI sites of the pAdApt.pac plasmid after removal of the fragment containing the pac linker. This resulting plasmid was named pAdApt.sal.

The Escherichia coli beta-Galactosidase (LacZ) transgene was amplified from the plasmid pMLP.nlsLacZ (EP 95-202 213) by PCR with the primers (5'-GGG GTG GCC AGG GTA CCT CTA GGC TTT TGC AA-3') and (5'-GGG GGG ATC CAT AAA CAA GTT CAG AAT CC-3'). The PCR product was digested with KpnI and BamHI and ligated into a KpnI/BamHI digested pcDNA3 plasmid (Invitrogen), giving rise to pcDNA3.nlsLacZ. This plasmid was digested with KpnI and BamHI. The resulting LacZ fragment was ligated to pAdApt.sal that was digested with the same enzymes. The resulting plasmid was named pAdApt.LacZ.sal.

pAC(d)CMVceNOS (transgene: human ce-Nitric Oxide Synthase, Janssens et al. 1998) was digested with EcoRI and the ends were filled in using Klenow enzyme. The ceNOS insert was removed by digestion with XbaI and isolated from gel. pAd5/CLIP was digested with BamHI and the ends were filled in by Klenow followed by digestion with XbaI. Ligation of the two fragments resulted in pAd5/CLIP.ceNOS. This plasmid was digested with HindIII and XbaI and the 3.7 kb ceNOS fragment was isolated and ligated to pAdApt.pac that was digested with the same enzymes. The resulting plasmid was named pAdApt.ceNOS.pac.

pAd5/CMV.rIL3beta (transgene: rat Interleukin-3, IL3, Esandi et al. 1998) was digested with HindIII and BamHI and the insert was ligated to pAd5/CLIP.sal which was digested with the same enzymes. pAdApt.pac was digested with BamHI and HindIII and the plasmid fragment was ligated to the rat IL3 insert that was isolated from pAd5/CLIP.rIL3, which was digested with the same enzymes. The resulting plasmid was named pAdApt.IL3.pac.

The pCMV.Luc plasmid (EP 95-202 213) plasmid (transgene: Luciferase) was digested with HindIII and BamHI and ligated to pAdApt.sal that was digested with the same enzymes, resulting in pAdApt.Luc.sal.

A 1231 nucleotide fragment of the human Smoothelin regulatory region located upstream of the coding region was obtained by PCR using an upstream (Smo-up-2) primer (5'-GAT CTA CGT AGT TAA CAG GCT GAA ACC ACC TCC CCA G-3') and a downstream (Smo-down) primer (5'-GAT CTA CGT ATC TCA GTC CAG CCC ACC CGT CCG-3') on plasmid #622 (described in example 4). pAdApt.ceNOS.pac and pAdApt.LacZ.sal are each digested with Acc65I and subsequently blunted by Klenow enzyme. The plasmids are then digested with SnaBI restriction enzyme and dephosphorylated by tSAP. The 1231 nucleotide Smoothelin HincII/BamHI PCR product is digested with SnaBI and ligated seperately to the digested pAdApt.ceNOS.pac and pAdApt.LacZ.sal to yield pAdSMO.ceNOS.pac and pAdSMO.LacZ.sal respectively.
The SnaBI digested 1231 nucleotide Smoothelin HincII/BamHI PCR product is also ligated seperately to pAdApt.IL3.pac and pAdApt.Luc.sal plasmids that are each digested with SnaBI and HindIII, blunted with Klenow and dephosphorylated by tSAP. The resulting plasmids are named pAdSMO.IL3.pac and pAdSMO.Luc.sal respectively.

### Example 7. Production of recombinant Adenoviruses.

pAdSMO.LacZ.sal and pAdSMO.Luc.sal are digested with SalI restriction enzyme to linearize the plasmids, while pAdSMO.ceNOS.pac and pAdSMO.IL3.pac are digested with PacI restriction enzyme. The plasmids are transfected individually together with a construct (pWE/Ad.AflII-rITR, ECACC deposit P97082116) carrying the right ITR and encoding the rest of the Adenoviral genome in PER.C6 cells and derivatives thereof using lipofectamine. Through homologous recombination in the overlapping Adenoviral DNA and by complementation of the Adenoviral E1 functions provided by the PER.C6cell line, recombinant Adenoviruses IG.Ad5.AdSMO.LacZ,

IG.Ad5.AdSMO.ceNOS, IG.Ad5.AdSMO.IL3 and IG.Ad5.AdSMO.Luc respectively are produced, propagated on PER.C6 cells and derivatives thereof and plaque purified. The respective viruses are purified to homogeneity by Cesium Chloride ultracentrifugation and virus particles are determined thereafter.

### Example 8. Infections in tissue culture cells.

Primary human Smooth Muscle Cells (SMCs), Human Umbilical Vein Endothelial Cells (HUVECs) and other primary cells are obtained from patient material. These and other cells (eg. PER.C6 and A549) are used for infections with the recombinant Adenoviruses carrying the 1231 nucleotide Smoothelin promoter driving the expression of the transgenes descibed in example 6 and 7. The human SMCs are also cultured in a way to induce a contractile phenotype.

Infections are performed with different multiplicities of infection. Cell lysates are obtained at different times after infection and tissue-specific transgene activities are determined using methods know to a person skilled in the art, depending on the transgene applied.

### Example 9. Intracoronary transgene expression in a porcine artery injury model.

Pigs are used as a percutaneous transluminal coronary angioplasty (PTCA) model and subsequently monitored to compare intracoronary and tissue specific transgene expression following infection with recombinant adenoviral vectors described in example 7 via the InfusaSleeve™ catheter in balloon angioplastied coronary arteries. Animals receive different doses of virus particles and will be sacrificed at different timepoints. Transgene expression will be monitored for tissue specific distribution in the layers of the arteries and for efficacy and toxicity studies in the treatment of restenosis as compared to non-tissue specific promoters driving the expression of the same transgenes.

Example 10. Pericardial delivery of Adenoviral vectors in pigs.
Transgene expression will be monitored after application of the recombinant Adenoviral vectors described in example 7 in using the pericardial space of pigs as a route for local drug administration to the heart and coronary arteries.
Administration of a recombinant adenoviral vector carrying the ceNOS transgene or the other transgenes described in example 6 into the pericardial space may result in encoded protein levels that are higher than obtained after administration of the virus directly into the injured vessel after balloon angioplasty.

Pigs are treated with different amounts of virus particles and monitored at different timepoints for efficacy, toxicity and tissue specificity of transgene distribution.

### Brief description of the drawings

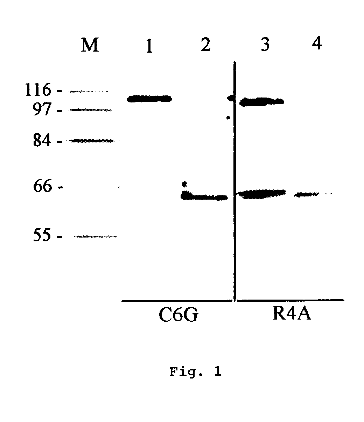

*FIG 1*. Two isoforms of smoothelin have been discovered. In vascular tissue a 110 kDa protein has been found, whereas a 59 kDa isoform has been found in smooth muscle tissue aligning the digestic tract and the urogenital system, as demonstrated by western blotting of human uterine artery (lanes 1 and 3) and human myoma tissue (lanes 2 and 4) with two smoothelin specific monoclonal antibodies R4A and C6G.

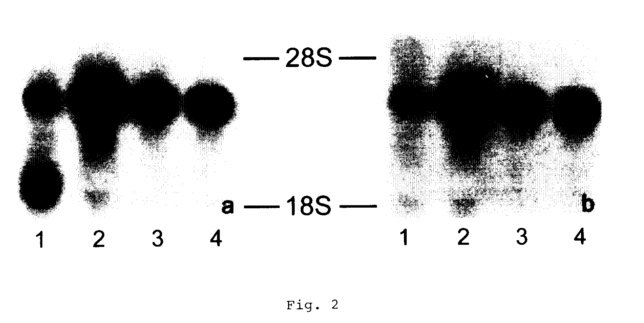

*FIG 2.*Northern blots of colon smooth muscle tissue (lane 1) and vascular smooth muscle cell cultures of respectively human umbelical cord, iliac artery and aorta (lanes 2 and 4) were hybridized with a probe containing exons 12-16 (panel a) and a probe containing exons 1-7 (panel b). Note that hybridization with the exons 12-16 probes in panel a, lane 1, reacts with both smoothelin mRNAs due to the presence of visceral mRNA and vascular mRNA derived from the blood vessels in the colon tissue.

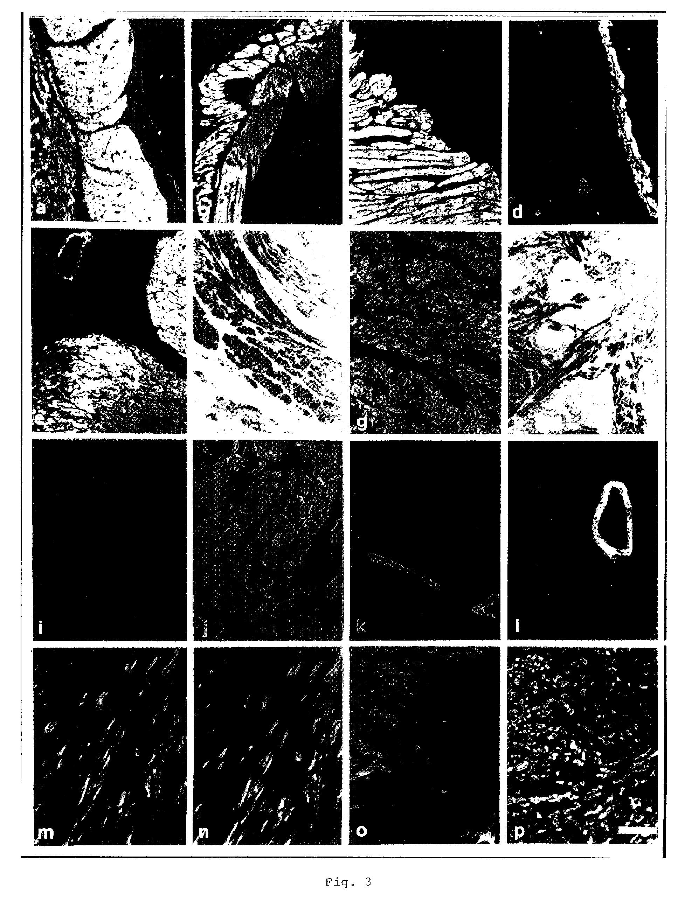

*FIG 3*.Immunohistochemical screening of different species and tissues for the presence of smoothelin. Smooth muscle tissue aligning the digestive tract of human (a), goat (b), chicken (c), and Xenopus laevis (d) all react with R$A. Human large intestine (e), stomach (f), uterus (g) and prostate (h) smooth muscle cells showed a strong reaction with R4A (f and h with peroxidase staining). In rabbit (k) and cat (l) heart tissue only smooth muscle cells of the bloodvessels are visualized by the anti-smoothelin monoclonal antibody. Double staining of the human heart (I and j) and of human myoma (m and n) with antibodies directed against smoothelin (I and m) and desmin (j and n) demonstrates the specificity for smooth muscle tissue of R4A, colocalization in myoma tissue but not in the heart. No colocalization of smoothelin (o) and vimentin (p) was found in sections of myoma.

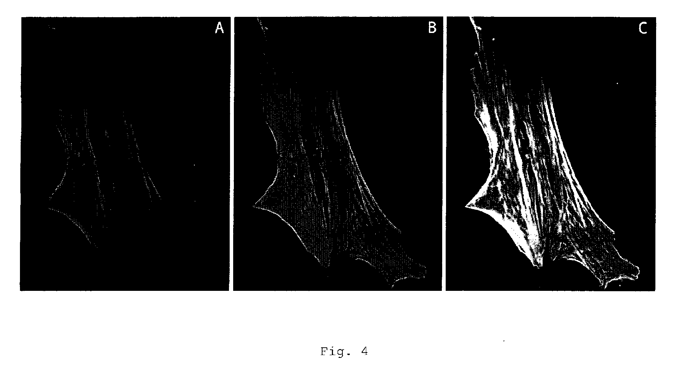

*FIG 4.* Smoothelin colocalizes largely with α-smooth muscle actin as demonstrated in primary cell cultures of porcine aorta derived smooth muscle cells. Confocal laser scanning microscopy images of vascular smooth muscle cells double stained for smoothelin (a) and α-smmoth muscle actin (b). Although areas exist where one of the two proteins displays a stronger presence, in general colocalization is significant as can be seen in the overlay.
*FIG 5.* Schematic representation of the smoothelin gene, the transcribed mRNAs and the encoded proteins. The upper line gives a restriction map of the genomic region that contains the smoothelin gene. (B:BamHI;
   H:HindIII;K:KpnI;X:XbaI)> The map of the gene conatins twenty exons represented by the broad grey blocks. The dot in the bars representing the proteins indicates the epitope for monoclonal antibody R$A.
*FIG 6.* 5' Regulatory region Human Smoothelin Genomic Clone
*FIG 7.* Nucleotide sequence of Exon 1 of the Human Smoothelin B gene.
*FIG 8.* Nucleotide sequence of Intron 1 of the Human Smoothelin B gene.
*FIG 9.* Nucleotide sequence of Exon 2 of the Human Smoothelin B gene.
*FIG 10.* Nucleotide sequence of the start of the intron 2 of the Human Smoothelin B gene.
*FIG 11.* Nucleotide sequence of Intron 8 of the Human Smoothelin B gene.
*FIG 12.* Nucleotide sequence of Exon 9 of the Human Smoothelin B gene.
*FIG 13.* Nucleotide sequence of Intron 9 of the Human Smoothelin B gene.
*FIG 14.* Nucleotide sequence of Exon 10 of the Human Smoothelin B gene.

### References

Altschul SF, Gish W, Miller W, Myers EW, Lipman DJ. Basic local alignment search tool. 1990. J. Mol. Biol. 215:403-410.

Auffray C, Rougeon F. Purification of mouse immunoglobulin heavy chain messenger RNAs from total myeloma tumor RNA. 1980. Eur. J. Biochem. 107:393-314.

Bauters C. Gene therapy for cardiovascular diseases. 1995. Heart J. 16:1166-1168.

Blank, R.S., McQuinn, T.C., Yin, K.C., Thompson, M.M., Takeyasu, K., Schwartz, R.J., Owens, G.K. 1992. Elements of the smooth muscle alpha-actin promoter required in cis for transcriptional activation in smooth muscle. Evidence for cell type-specific regulation. J. Biol. Chem. 267 (2):984-989

Boshart et al. (1985) A very strong enhancer is located upstream of an immediate early gene of human cytomegalovirus. Cell 41: 521-530

Campbell JH, Kocher O, Skalli O, Gabbiani G, Campbell GR. Cytodifferentiation and expression of alpha-smooth muscle actin: a new probe for smooth muscle differentiation. 1989. Atherosclerosis. 9:633-643.

Carroll, S.L., Bergsma, D.J., Schwartz, R.J. 1986. Structure and complete nucleotide sequence of the chicken alpha-smooth muscle (aortic) actin gene. An actin gene which produces multiple messenger RNAs. J. Biol. Chem. 261 (19): 8965-8976

Christ B, Ordahl CP. Early stages of chick somite development. 1995. Anat. Embryol. 191:381-396.

Church GM, Gilbert W. Genomic sequencing. 1984. Proc. Natl. Acad. Sci. USA 81:1991-1995.

Engelen JJM, Esterling LE, Albrechts JCM, Detera-Wadleigh SD, Van Eys GJJM. Assignment of the human gene for smoothelin (SMTN) to chromosome 22q12 by fluorescence in situ hybridisation and radiation mapping. 1997. Genomics 43:245-247.

Esandi et al. (1998) Cloning, Biological characterization and high level expression of rat Interleukin-3 using recombinant Adenovirus, description of a new splicing variant. Gene 211: 151-158

Feinberg AP, Vogelstein B. A technique for radiolabeling DNA restriction endonuclease fragments to high specific activity. 1983. Anal. Biochem. 132:6-12.

Gabbiani G, Schmid E, Winter S, Chaponnier C, de Chastonay C, Venderkerckhove J, Weber K, Franke WW. Vascular smooth muscle cells differ from other smooth muscle cells: predominance of vimentin filaments and a specific alpha-type actin. 1981. Proc. Natl. Acad. Sci. USA. 78:298-302.

Geourjon C, Deleage G. Sopma: a self optimized prediction method for protein secondary structure prediction. 1994. Prot. Eng. 7:157-164.

Geourjon C, Deleage G. Sopma: significant improvements in protein secondary structure prediction by prediction from multiple alignments. 1995. Comput. Applic. Biosci. 11:681-684.

Gilbert SF. Developmental Biology. 1991. 3th edition:202. Sinauer Ass. Inc. Sunderland, MA USA.

Haeberle JR, Hathaway DR, Smith CL. Caldesmon content of mammalian smooth muscles. 1992. J. Muscle Res. Cell Motil. 13:81-89.

Herring BP, Smith AF. Telokin expression is mediated by a smooth muscle cell-specific promoter. 1996. Am. J. Physiol. 270:C1656-1665.

Janssens et al. (1998) Human endothelial nitric oxide synthase gene transfer inhibits vascular smooth muscle cell proliferation and neointima formation after balloon injury in rats. Circulation 97: 1274-1281

Kay et al. (1990) Expression cloning of a cDNA encoding M1/69. J. Immunol. 145: 1952-1959

Kemp PR, Osbourn JK, Grainger DJ, Metcalfe JC. Cloning and analysis of the promoter region of the rat SM22alpha gene. 1995. Biochem. J. 310:1037-1043.

Kim S, Lin H, Barr E, Chu L, Leiden JM, Parmacek MS. Transcriptional targeting of replication-defective adenovirus transgene expression to smooth muscle cells in vivo. 1997. J. Clin. Invest. 100:1006-1014.

Köhler G, Milstein C. Continuous cultures of fused cells secreting antibody of predefined specificity. 1975 Nature 256:495-495.

Laemmli UK. Cleavage of structural proteins during the assembly of the heads of the bacteriophage T4. 1970. Nature 227:680-685.

Levrero, M., Barban, V., Manteca, S., Ballay, A., Balsamo, C., Avantaggiata, M.L., Natoli, G., Skellekens, H., Tiollais, P., and Perricaudet, M. (1991). Defective and nondefective adenovirus vectors for expression foreign genes in vitro and in vivo. Gene 101, 195-202.

Li L, Miano JM, Mercer B, Olson EN. Expression of SM22alpha promoter in transgenic mice provides evidence for distinct transcriptional regulatory programs in vascular and visceral smooth muscle cells. 1996a. J. Cell Biol. 132:849-859.

Li L, Miano JM, Cserjesi P, Olson EN. SM22 alpha, a marker of adult smooth muscle, is expressed in multiple myogenic lineages during embryogenesis. 1996b. Circ. Res. 78:188-195.

Mack, C.P., Owens, G.K. Regulation of smooth muscle alpha-actin expression in vivo is dependent on CarG elements within the 5' and first intron promoter regions. 1999. Circ. Res. 84 (7): 852-861

Madsen, C.S., Regan, C.P., Hungerford, J.E., White, S.L., Manabe, I., Owens, G.K. Smooth muscle-specific expression of the smooth muscle myosin heavy chain gene in transgeneic mice requires 5'-flanking and first intronic DNA sequence. 1989. Circ. Res. 82 (8): 908-917

Mano, T., Luo, Z., Malendowicz, S.L., Evans, T., Walsh, K. 1999. Reversal of GATA-6 downregulation promotes smooth muscle differentiation and inhibits intimal hyperplasia in balloon-injured rat carotid artery. Circ. Res. 84 (6): 647-654

Min, B.H., Foster, D.N., Strauch, A.R. 1990. The 5'-flanking region of the mouse vascular smooth muscle alpha-actin gene contains evolutionary conserved sequence motifs within a functional promoter. J. Biol. Chem. 265 (27):16667-16675

Nagai, R., Kuro-O, M., Babij, P., Periassamy, M. (1989). Identification of two types of smooth muscle myosin heavy chain isoforms by cDNA cloning and immunoblot analysis. J. Biol. Chem. 264, 9734-9737

Nikol S, Huehns TY, Hofling B. Molecular biology and post-angioplasty restenosis. 1996. Atherosclerosis 123:17-31.

Owens GK. Regulation of differentiation of vascular smooth muscle cells. 1995. Physiol. Rev. 75:487-517.

Ramaekers FCS, Moesker, 0, Huijsmans A, Schaart G, Westerhof G, Wagenaar Sj Sc, Herman CJ, Vooijs GP. Intermediate filament proteins in the study oftumor heterogeneity: an in-depth study of tumors of the urinary and respiratory tracts. 1985. Ann. NY Acad. Sci 455:614-634.

Reddy, S., Ozgur, K., Lu, M., Chang, W., Mohan, S.R., Kumar, C.C., Ruley, H.E. 1990. Structure of the human smooth muscle alpha-actin gene. Analysis of a cDNA and 5' upstream region. J. Biol. Chem.265 (3):1683-1687

Ross R. The pathogenesis of atherosclerosis: a perspective for the 1990s. 1993. Nature 362:801-809.

Rost B, Sander C. Prediction of protein secondary structure at better than 70% accuracy. 1993. J. Mol. Biol. 232:584-599.

Rost B, Sander C. Combining evolutionary information and neural networks tp predict protein secondary structure. 1994. Proteins. 19:55-77

De Ruiter MC, Poelman RE, van Munsteren, JC, Mironov, V, Mark-Wald RR, Gittenberger-de Groot AC. Embryonic endothelial cells transdifferentiate into mesenchymal cells expressing smooth muscle actins in vivo and in vitro. Circ. Res. 1997. 80:444-451.

Sambrook J, Fritsch EF, Maniatis T. Molecular cloning: A laboratory manual. 1989. Cold Spring Harbor, New York: Cold Spring Harbor Laboratory Press.

Shanahan CM, Weisberg PL, Metcalfe JC. Isolation of gene markers of differentiated and proliferating vascular smooth muscle cells. 1993. Circ. Res. 73:193-204.

Shanahan CM, Weissberg PL. Smooth muscle cell heterogeneity. Patterns of gene expression in vascular smooth muscle cells in vitro and in vivo. 1998. Arterioscler. Thromb. Vasc. Biol. 18:333-338.

Skalli 0, Ropraz P, Trzeciak A, Benzonana G, Gillesen G, Gabbiani G. A monoclonal antibody against alpha-smooth actin: a new probe for smooth muscle differentiation. 1986. J. Cell Biol. 103: 2787-2796.

Smith AF, Bigsby RM, Word RA, Herring BP. A 310-bp minimal promoter mediates smooth muscle cell-specific expression of telokin. 1998. Am. J. Physiol. 274:C1188-C1195.

Takahashi, K., Nadal-Ginard, B. (1991) Molecular cloning and sequence analysis of smooth muscle calponier. J. Biol. Chem 266, 13284-13288.
Van der Loop FTL, Schaart G, Timmer EDJ, Ramakers FCS, Van Eys GJJM. Smoothelin, a novel cytoskeletal protein specific for smooth muscle cells. 1996. J. Cell Biol. 134:401-411.

Van Eys GJJM, Voller MCW, Timmer EDJ, Wehrens XHT, Small JV, Schalken JA, Ramaekers FCS, Van der Loop FTL. Smoothelin expression characteristics: development of a smooth muscle cell in vitro system and identification of a vascular variant. 1997. Cell Struct. Funct. 22:65-72.

Van Eys, GJJM, De Vries, CJM, Rensen, SSM, Thijssen, VLJL, Verkaar, ELC, Coolen, GPGM, Debie, WMH, de Ruiter, MC, Wadleigh-Detera, SD. (1999). Smoothelins: One gene, two proteins, three muscle cell types..so far. In: Cardiovascular specific gene expression. Pp 49-66 Eds. PA Doevendans, RS Reneman, M van Bilsen. Kluwer Acad Publ. Dortrecht/ Boston/London

Wehrens XHT, Mies G, Gimona M, Ramaekers FCS, Van Eys GJJM, Small JV. Substructural localization of smoothelin and a vascular specific variant. 1997. FEBS 405:315-320.

Yablonka-Reuveni Z, Schwartz SM, Christ B. Development of chicken aortic smooth muscle: Expression of cytoskeletal and basement membrane proteins defines two distinct cell phenotypes emerging from a common lineage. 1993. Cell. Mol. Biol. Res. 41:241-249.

Yamamura H, Masuda H, Ikeda W, Tokuyama T, Takagi M, Shibata N, Tatsuta M, Takahashi K. Structure and expression of the human SM22alpha gene, assignment of the gene to chromosome 11, and repression of the promoter activity by cytosine DNA methylation. 1997. J. Biochem. (Tokyo) 122:157-167.

Yet SF, Folta SC, Jain MK, Hsieh CM, Maemura K, Layne MD, Zhang D, Marria PB, Yoshizumi M, Chin MT, Perrella MA, Lee ME. Molecular cloning, characterization, and promoter analysis of the mouse Crp2/SmLim gene. Preferential expression of its promoter in the vascular smooth muscle cells of transgenic mice. 1998. J. Biol. Chem. 273:10530-10537.

**Table 1:**

| 5'splice site | exon | size | 3'splice site | intron size |
|---|---|---|---|---|
| | 1 | 138 | TGAGAGgtgggt | 1760 |
| ctgcagAATTCT | 2 | 131 | AAGCTGgtaagt | 4550 |
| ctgcagCTGGAG | 3 | 149 | GCTGCAgtgagt | 400 |
| ctgcagCTCTCA | 4 | 94 | GCACTGgtgagg | 90 |
| ccacagTTGCGA | 5 | 79 | TTGAGGgtatgt | 100 |
| ttccagCTGCCA | 6 | 98 | TGTGAGgtaagg | 660 |
| ttacagGTGCCA | 7 | 323 | AACAAGgtgagt | 90 |
| ttgcagCTTCTG | 8 | 73 | GAGCAGgtgagg | 510 |
| tgccagACGTGG | 9 | 72 | ACCGAGgtacta | 80 |
| ttctagAGTCCA | 10 | 522 | GAGGGCgtaggc | 192 |
| actcagAACTGA | 11 | 173 | ATCAAGgtgagc | 3000 |
| ctgcagATGGAA | 12 | 150 | AAGATGgtatag | 80 |
| atgcagCTGGAT | 13 | 75 | AGAGAGgtagag | 1200 |
| gcccagACCAGC | 14 | 163 | ACTCCAgtaagg | 103 |
| caggagGCACCA | 15 | 42 | CGGAGAgtaagg | 203 |
| ttgcagATGGCA | 16 | 70 | GGGCAGgtgagc | 2000 |
| ccctagCATCTT | 17 | 175 | GCCCGCgtgagc | 221 |
| tacagcAGCCCT | 18 | 117 | TACGAGgtgagc | 550 |
| tagtaaGTCGAC | 19 | 149 | TGCGAAgtaagt | 2100 |
| ctgcagGATGCT | 20 | 309 | | |
| Table 1: Presentation of intron-exon splice recognition sequences and the size of exons and introns. | | | | |

## Claims

1. A nucleic acid delivery vehicle comprising nucleic acid of interest capable of expressing specifically in a contractile smooth muscle cell.

2. A nucleic acid delivery vehicle according to claim 1, wherein said nucleic acid of interest is capable of expressing specifically in a vascular contractile smooth muscle cell and/or a visceral contractile smooth muscle cell.

3. A nucleic acid delivery vehicle according to claim 1 or claim 2, comprising a smoothelin gene promoter, or a functional part, derivative and/or analogue thereof.

4. A nucleic acid delivery vehicle according to claim 3, wherein said smoothelin gene is derived from a human.

5. A nucleic acid delivery vehicle according to claim 3 or claim 4, wherein said promoter is the promoter driving smoothelin B expression, or a functional part, derivative and/or analogue thereof.

6. A nucleic acid delivery vehicle to anyone of claim 3-5, wherein said promoter comprises a sequence as depicted in anyone of figures 6-14, or a functional part, derivative and/or analogue thereof.

7. A nucleic acid delivery vehicle according to anyone of claims 1-6, comprising a virus-like particle.

8. A nucleic acid delivery vehicle according to claim 7, wherein said virus-like particle is an adenovirus particle, an adeno-associated virus particle or a retrovirus particle, or a functional part, derivative and/or analogue thereof.

9. A nucleic acid delivery vehicle according to anyone of claims 1-8, comprising a tissue tropism determining part of an adenovirus of subgroup B, D and/or F, or a functional part, derivative and/or analogue thereof.

10. A nucleic acid delivery vehicle according to anyone of claims 7-9, wherein said nucleic acid delivery vehicle comprises an adenovirus particle comprising nucleic acid derived from adenovirus.

11. A nucleic acid delivery vehicle according to claim 10, wherein said nucleic acid derived from adenovirus comprises a functional deletion of an adenovirus early region 1 encoded protein.

12. A nucleic acid delivery vehicle according to claim 10 or claim 11, wherein said nucleic acid derived from adenovirus comprises a functional deletion of an adenovirus early region 2 and/or early region 4 encoded protein.

13. A nucleic acid delivery vehicle according to anyone of claims 10-12, wherein said nucleic acid derived from adenovirus comprises a minimal adenovirus vector or an Ad/AAV chimeric vector.

14. A nucleic acid delivery vehicle according to anyone of the claims 1-13, further comprising a nucleic acid of interest.

15. A nucleic acid delivery vehicle according anyone of claims 1-14, comprising a nucleic acid encoding a nitric oxide synthetase and/or a GATA6, or a functional part, derivative and/or analogue thereof.

16. A nucleic acid delivery vehicle according to anyone of claims 1-15, comprising at least one of the adenovirus serotype 35 elements or a functional equivalent thereof, responsible for avoiding or diminishing neutralising activity against adenoviral elements by the host to which the nucleic acid on interest is to be delivered.

17. A cell provided with nucleic acid of interest capable of expressing specifically in a contractile smooth muscle cell.

18. A cell according to claim 17, wherein said cell is a contractile smooth muscle cell.

19. A cell comprising a nucleic acid delivery vehicle according to anyone of claims 1-16.

20. A cell according to claim 19, capable of producing said nucleic acid delivery vehicle.

21. A cell according to anyone of claims 17-20, comprising nucleic acid encoding an adenovirus early region 1 encoded protein, or a functional part, derivative and/or analogue thereof.

22. A cell according to anyone of claims 17-21, comprising nucleic acid encoding an adenovirus early region 2 and/or an adenovirus early region 4 encoded protein, or a functional part, derivative and/or analogue thereof.

23. A cell according to anyone of claims 17-22, wherein said cell is a PER.C6 cell (ECCAC deposit number 96022940) or a derivative thereof.

24. A method for providing a cell with a nucleic acid of interest capable of expressing specifically in a contractile smooth muscle cell comprising contacting said cell or a precursor thereof with a nucleic acid delivery vehicle according to anyone of claims 1-16.

25. Use of a nucleic acid delivery vehicle according to anyone of claims 1-16, for the preparation of a pharmaceutical.

26. Use of a nucleic acid delivery vehicle according to anyone of claims 1-16, for the preparation of a pharmaceutical for the treatment of a cardiovascular disease.

27. A promoter of a smoothelin gene, or a functional part, derivative and/or analogue thereof.

28. A promoter according to claim 27, comprising a sequence as depicted in anyone of figures 6-14, or a functional part, derivative and/or analogue thereof.

29. Use of a promoter, or a functional part, derivative and/or analogue thereof of a smoothelin gene, for providing a nucleic acid of interest with the capacity to express specifically in a contractile smooth muscle cell.

30. A use according to claim 29, wherein said promoter is a promoter according to claim 27 or claim 28.

31. A nucleic acid as depicted in figure 6-14, or a functional equivalent thereof.

32. A method for the phenotypic characterisation of a cell comprising providing said cell with a nucleic acid delivery vehicle according to anyone of claims 1-16, culturing said cell to allow cell type specific expression of said nucleic acid of interest, measuring whether said nucleic acid is expressed in said cell and inferring from said measurement the phenotype of said cell.


### Annex to the application documents - subsequently filed sequences listing

SEQUENCE LISTING

<110> Introgene B.V.

<120> Smooth muscle cell promoter and uses thereof

<130> P50241EP00

<140> 99202943.9
<141> 1999-09-09

<160> 61

<170> PatentIn Ver. 2.1

<210> 1
<211> 47
<212> DNA
<213> Artificial Sequence

<220>
<223> Description of Artificial Sequence: LTR-1

<220>
<221> primer_bind
<222> (1)..(47)

<400> 1
ctgtacgtac cagtgcactg gcctaggcat ggaaaaatac ataactg 47

<210> 2
<211> 64
<212> DNA
<213> Artificial Sequence

<220>
<223> Description of Artificial Sequence: LTR-2

<220>
<221> primer_bind
<222> (1)..(64)

<400> 2
gcggatcctt cgaaccatgg taagcttggt accgctagcg ttaaccgggc gactcagtca 60
atcg 64

<210> 3
<211> 28
<212> DNA
<213> Artificial Sequence

<220>
<223> Description of Artificial Sequence: HSA1

<220>
<221> primer_bind
<222> (1)..(28)

<400> 3

gcgccaccat gggcagagcg atggtggc                                    28

<210> 4
<211> 50
<212> DNA
<213> Artificial Sequence

<220>
<223> Description of Artificial Sequence: HSA2

<220>
<221> primer_bind
<222> (1)..(50)

<400> 4
gttagatcta agcttgtcga catcgatcta ctaacagtag agatgtagaa          50

<210> 5
<211> 10
<212> DNA
<213> Artificial Sequence

<220>
<223> Description of Artificial Sequence: oligo

<220>
<221> misc_feature
<222> (1)..(10)

<400> 5
ttaagtcgac                                                      10

<210> 6
<211> 23
<212> DNA
<213> Artificial Sequence

<220>
<223> Description of Artificial Sequence: linker

<220>
<221> misc_feature
<222> (1)..(23)

<400> 6
aattgtctta attaaccgct taa                                       23

<210> 7
<211> 67
<212> DNA
<213> Artificial Sequence

<220>
<223> Description of Artificial Sequence: PLL-1

<220>
<221> primer_bind

<222> (1)..(67)

<400> 7
gccatcccta ggaagcttgg taccggtgaa ttcgctagcg ttaacggatc ctctagacga 60
gatctgg 67

<210> 8
<211> 67
<212> DNA
<213> Artificial Sequence

<220>
<223> Description of Artificial Sequence: PLL-2

<220>
<221> primer_bind
<222> (1)..(67)

<400> 8
ccagatctcg tctagaggat ccgttaacgc tagcgaattc accggtacca agcttcctag 60
ggatggc 67

<210> 9
<211> 39
<212> DNA
<213> Artificial Sequence

<220>
<223> Description of Artificial Sequence: CMVplus

<220>
<221> primer_bind
<222> (1)..(39)

<400> 9
gatcggtacc actgcagtgg tcaatattgg ccattagcc 39

<210> 10
<211> 29
<212> DNA
<213> Artificial Sequence

<220>
<223> Description of Artificial Sequence: CMVminA

<220>
<221> primer_bind
<222> (1)..(29)

<400> 10
gatcaagctt ccaatgcacc gttcccggc 29

<210> 11
<211> 32
<212> DNA
<213> Artificial Sequence

<220>
<223> Description of Artificial Sequence: primer

<220>
<221> primer_bind
<222> (1)..(32)

<400> 11
ggggtggcca gggtacctct aggcttttgc aa                                    32


<210> 12
<211> 29
<212> DNA
<213> Artificial Sequence

<220>
<223> Description of Artificial Sequence: primer

<220>
<221> primer_bind
<222> (1)..(29)

<400> 12
   ggggggatcc ataaacaagt tcagaatcc                                         29


<210> 13
<211> 37
<212> DNA
<213> Artificial Sequence

<220>
<223> Description of Artificial Sequence: Smo-up-2 primer

<220>
<221> primer_bind
<222> (1)..(37)

<400> 13
gatctacgta gttaacaggc tgaaaccacc tccccag                               37


<210> 14
<211> 33
<212> DNA
<213> Artificial Sequence

<220>
<223> Description of Artificial Sequence: Smo-down primer

<220>
<221> primer_bind
<222> (1)..(33)

<400> 14
gatctacgta tctcagtcca gcccacccgt ccg                                   33


<210> 15
<211> 12

<212> DNA
<213> Artificial Sequence

<220>
<223> Description of Artificial Sequence: variant intron-exon splice recognition
sequences

<220>
<221> misc_feature
<222> (1)..(12)

<400> 15
ctgcagaatt ct                                                          12


<210> 16
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 16
ctgcagctgg ag                                                          12


<210> 17
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 17
ctgcagctct ca                                                          12


<210> 18
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 18
ccacagttgc ga                                                          12


<210> 19
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 19
ttccagctgc ca                                                          12


<210> 20
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 20
ttacaggtgc ca                                                          12


<210> 21
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 21
ttgcagcttc tg                                                          12


<210> 22
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 22
tgccagacgt gg                                                          12


<210> 23
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 23
ttctagagtc ca                                                          12


<210> 24
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 24
actcagaact ga                                                          12


<210> 25
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 25
ctgcagatgg aa                                                          12

<210> 26
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 26
atgcagctgg at                                                          12


<210> 27
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 27
gcccagacca gc                                                          12


<210> 28
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 28
caggaggcac ca                                                          12


<210> 29
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 29
ttgcagatgg ca                                                          12


<210> 30
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 30
ccctagcatc tt                                                          12


<210> 31
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 31
tacagcagcc ct                                                          12


<210> 32
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 32
tagtaagtcg ac                                                          12


<210> 33

<211> 12
<212> DNA
<213> Artificial Sequence

<220>
<223> Description of Artificial Sequence: variant intron-exon splice recognition
sequences

<220>
<221> misc_feature
<222> (1)..(12)

<400> 33
ctgcaggatg ct                                                          12


<210> 34
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 34
tgagaggtgg gt                                                          12


<210> 35
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 35
aagctggtaa gt                                                          12


<210> 36
<211> 12
<212> DNA
<213> Artificial Sequence

<220>
<223> Description of Artificial Sequence: variant intron-exon splice recognition
sequences

<220>
<221> misc_feature

<222> (1)..(12)

<400> 36
gctgcagtga gt                                                          12


<210> 37
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 37
gcactggtga gg                                                          12


<210> 38
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 38
ttgagggtat gt                                                          12


<210> 39
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 39
tgtgaggtaa gg                                                          12


<210> 40
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 40
aacaaggtga gt                                                          12


<210> 41
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 41
gagcaggtga gg                                                          12


<210> 42
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 42
accgaggtac ta                                                          12


<210> 43
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 43

gagggcgtag gc                                                          12


<210> 44
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 44
atcaaggtga gc                                                          12


<210> 45
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 45
aagatggtat ag                                                          12


<210> 46
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 46
agagaggtag ag                                                          12


<210> 47
<211> 12
<212> DNA
<213> Artificial Sequence

<220>

<223> Description of Artificial Sequence: variant intron-exon splice recognition
sequences

<220>
<221> misc_feature
<222> (1)..(12)

<400> 47
actccagtaa gg                                                          12


<210> 48
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 48
cggagagtaa gg                                                          12


<210> 49
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 49
gggcaggtga gc                                                          12


<210> 50
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 50
gcccgcgtga gc                                                          12

<210> 51
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 51
tacgaggtga gc                                                          12


<210> 52
<211> 12
<212> DNA
<213> Artificial Sequence

<220>
<223> Description of Artificial Sequence: variant intron-exon splice recognition
sequences

<220>
<221> misc_feature
<222> (1)..(12)

<400> 52
tgcgaagtaa gt                                                          12


<210> 53
<211> 3369
<212> DNA
<213> human

<220>
<221> promoter
<222> (1)..(3369)
<223> /note="5' Regulatory region human smoothelin
      genomic clone"

<400> 53
ggatccagcc aaccccaaca agaaccatcc acctctaggg gctccattct ccagcctcac 60
ctgcctgtct gttgcccaac aaggtcgtca caatcctact tccgttcctt tgctaatgct 120
gttcccgtct ccagaatgac agtgctgtcc tggggagaga ggcttagaga gcccaaggca 180
cagctcctcc attgtgcata tgagcaagtt gaagctccaa gagggccaac ccttacctaa 240
ggttggagcc aggggtaaga cacccatcac ccttcttgcc tcctgaccca aggcctgggc 300
tttctctgaa ggacctgact gggtggaggg ttaccagggt gacttaggga tgcaacacag 360
acagcttggt aaaatagtca ttctcagact tgagctctgg agcaaaatat tacccctagc 420
ctctgagctg ctccagccac ttcccattct gcctttccag ggttgaacct ggaagaacct 480
agagtagacc ctttctgtga cagccccttg ccctaggaag gcttgaagca gaccagtcta 540
atcacctcat tttacagggg gcaggctgag gcccaggcct ctctggtgcc cacaagatac 600
attcatcctg ctcatctggc attcagaatc cttggcaaag agaaccaccc acatttcctg 660
ctccgcccac agcctatcct cctccttttc ccagactatt ggtctttgct cacactgttc 720
ctctgccaca aatgcccttt catgcctttc cccctcttct atgtgggatg aattttaaaa 780
attagctggg cgtggtggtg tgtgcctgta gtcctagcct cttgggaggc tgagatggaa 840

ggattgcttg agccaggagt ttgaggccat ggtaagctgt gatcatgcga ctgcactcca 900
gcctgtggga cacagcaaga ccctgtctcc aaaaaaaata aaataaaata aaaacaagtg 960
tgacacacag tttatacacc acttccctaa gaagtcttcc agagcttccc ccatcctagt 1020
ggcagcaaag tggagggaag gggcttcagc atcaggcaga attccacttt agtctgaatg 1080
cctttactag gccaaggtgc cttcacctcc ctgagcttca gcttcctctg tcgaatcaga 1140
gtgtacctat cttgcacagt ggtcacctgg cagtagtggg agccccacgt ctgacatgca 1200
acaggtgttc agcaaatgct ggggccctcg tcacgggatg tttactcttt cctcggctgt 1260
agctgtaaac ttccctagca ctgtaccgga ctgccctttg atctctagat cacccccact 1320
cccttgcaga gaggaagaca gaggcaggcg ggtatgtctg gaggtggacc caggcactaa 1380
gggcttgaag gggcaggacc cacacatccc ttccataggg ccaagggcta gtgagcgcat 1440
gtgccagaca gtaaatccaa gcatggagga actcacctgt accattcctc ctagcctaca 1500
gtagggcagg gagaaaggag ggcattagca caaagcaggc cattcattgg agctggtgac 1560
cgccattcag gcaggccctg aaaatcagag gtggcctagg tgtgccctgc atgaagggga 1620
ggtcccaagg gacgcgtgaa ttttgaagta ttcagaattg gtcttagagg gcgcctgacc 1680
ctcgttctgg ataataacat ggcatctcgc ccaggtagag aaagcctagg accttgaaat 1740
atgtcctccg aagtgagctc tgtttgcctc tcctgctgtg tagcattgac aagtcgcttt 1800
cttctgtgcc ttgattttct tttttctttt tttgagacag aatcttgctc tgtcgcttag 1860
gctggagtgc agtggcacaa tctcggctca ctgcaacctc tgcctccagg ttcaagtgat 1920
tctactgcct cagcctccca agtagctggg attacaggtg catgccactt gcccgactaa 1980
tttttgtatt tttagtagag acggggtttt tgccgtgttg gccaggctgg tctcgaactc 2040
ctgaccttgg gtgatccacc tgccttggca tcccaaagtg ctgggattac aggcatgagc 2100
caccgcacct ggcccgtgcc ttggttttct gatgtgtaaa atgggaccac ttctcagggg 2160
tgtggggatg aaatgaaatg acacatggaa aagactctca gtgtggagac tgggcctttc 2220
cttagatggg ggctgggggg tgtcacagag gacctgaacc ggggctcttc tgggttaaca 2280
ggctgaaacc acctccccag aatcccacat tatagtggtt aaaagcaggg tctggactca 2340
gaaggtgctg ggtatgaatc acagctttac tacttatcag ctgttgtcct catctgtaaa 2400
atggggcaat agtcatggtc tcatgtgctt gttatgggca ttatatgcct ggtacatggc 2460
aagtacaaaa taaatgattt gttattgttt ttatttgaca ggactccaga ccccatcctc 2520
ccaactccac ccactttagt ctccatggag acccaagggc ggctttaggg cctctggcac 2580
tgaagatgga atagctttgc cttgtggatc tgacctcctg ggtacctggc tgattccttg 2640
tttggtcttg gacatgtcag ctctctctgg gcctcaattt cccccttttg ctgaatgaca 2700
gcctggactc catattccct atttaggact ccagcgctcc taaaggcaga ctctcagggc 2760
ttggaaggta gaagcatgct acaagaaggc aatgtctgtt caaaggtgtg tgggctgggg 2820
gaggcgtgct gtgtgattga ttctgctcca cctgagatgg tcccggcact ccaacacagg 2880
gtcaaggtgc tgccagtcac tcagtccctg ctctgaggtg gctcagacca ggtgtcccga 2940
aagcagccta gggacggcgg ccaagcttaa acactgacgc gagaaagggg tggagtcgag 3000
ggaggggacg tggtctcagg aacaaagagg agggcggaga ggggctgtcc tgaaggctct 3060
gagactccta cgaccccagg aaacttacct cggctcgggg cgcccccccc gacttggcgc 3120
agagcctgcg tccccctgcc gatgcccacc cgactcgcta aagccaccga ggcagacgac 3180
cccaagactc ccaatttccg acctccctgg cccgcgaggg gcggggcctt cctctcgggg 3240
cgtggccgtc aaattgaatt ttcccaatgg ggcacgagag tgggaggtac ccaatcgagg 3300
gttggctggc gcgtcggggc agggcggggc cagccgggct gctggcgcgg ctggacgggc 3360
agctctccg 3369

<210> 54
<211> 138
<212> DNA
<213> human

<220>
<221> misc_feature
<222> (1)..(138)
<223> /note="Exon 1"

<400> 54
cagaatccag gggacggttg ctgagcgggc ctgggacagc gggtcgcggc acctccggcc 60
tgcgcgtgtc taatccgtct gtcgggtccc gaaagagcta agccgagcct gcgccggacg 120
ggtgggctgg actgagag 138

<210> 55
<211> 1722
<212> DNA
<213> human

<220>
<221> misc_feature
<222> (1)..(1722)
<223> /note="Intron 1"

<400> 55
gtgggtgtgc ggacctcggg ggggatcctg gtgctgcccc aacgtgtccg ggggtggaac 60
ccggatgtct agacaggagt tctggaacgt ccagacctgg gagcccccac ccatacgaga 120
ggtctaagcc atgggataaa gttatgagac tgttggggga agacgccccc ggattcctaa 180
gagactccac gccccacagg ggcgctagtc tgtggcggca gaaaccctgg ggaagctgcc 240
acagaatcct gaggcccctc acaatgtccg gcgaccccag actgtgtgac agggagattg 300
gagaagagca gcggcaggga tcttcttata atcccatggg tgtcctagat taggcattag 360
ggtcagctgc cccatagaag aacagacatg gggggaatca gaatgtccgc agaacctaag 420
gctggtgacc actggacagt cttgaagagg tttaacccag acagagtaac taggtaagag 480
gcaaggatcc taacaggcag agattgggag gttgacacct ctgagcctac caggatggga 540
gggggaacaa gcagtgactt gaacaggctg ggacccacac accccaactg tgtggagcgg 600
taaccctcac cccccaccca tgcaccgtat ggggtgactt cccaagtgac tgttctgaag 660
agagggaagg gatgattgaa agatgaatca acaggaggtg gggggcaggc ctggggccac 720
acattagtca gtcaccatac attaactaag catccacagt gtgccagacc agggcccctc 780
aaattccaga cagacattag ttggggctac aggaggaagg acagatctgc gctgggaggc 840
tgggccagga tttaggggtc cattttgatg ggcagcactg gggcaagatg gggctcttgc 900
ctctgtcact gagagtgttc caggcccttg agggcacttt ggaagggagc ttcggaagcc 960
ggagatgacg aagattttga ttacatcctt ctctgccctc taagtctttg gcttcctcct 1020
tgctcagtga caaatccttg gccctctcca atcttctcag tttgcctgaa atccaggggt 1080
tgggcaggtc agcttctgaa agcccttcca ggtggggaat tgaattctgt atgaattggc 1140
aggaggtgct gggtgcctgg acacctggct tcaggaggtc tggacagcag agctggctcc 1200
aagaatcgga agacacagtg taccttccta tacctggggg aatgacagtg gtggtggcag 1260
caagaactac gggttcagcc cacagccaga agctgagcct gcggagtggg tgggggctgg 1320
gtaggccctg tggatgggtg ggtggggcag gaaactggag actggagtgc accccctggt 1380
ggtaagcaca gcttgggtgg gtcttgccag gccctggctt ctcaatccag gtccctgccc 1440
tccctgtttt gaggtttaga caggatgaga gtccatccca ggccacacag cagactggcg 1500
gccaagctgg cagggctgga accagagacc ccctaccctg ggtttggtga gttttccagc 1560
aaaccaccct agggcagagg gcagcttcca gccaggagcc acattatagg atgggacagt 1620
aatggacgct cctaggttag agagggaaag taaggcacag agcctaagtg cctgtggttt 1680
ctggaagccc cagcatgcct gattcatgtc tgtcccctgc ag 1722


<210> 56
<211> 131
<212> DNA
<213> human

<220>
<221> misc_feature
<222> (1)..(131)
<223> /note="Exon 2"

<400> 56
aattctctga gctggtgaca ggtgccacag gcactgggga tctcaccaga aaggaaccga 60
cggagctagg ggccagcgag atggcggacg aggccttagc tgggctggat gagggagccc 120
ttcggaagct g 131


<210> 57
<211> 160
<212> DNA

<213> human

<220>
<221> misc_feature
<222> (1)..(160)
<223> /note="Start intron 2"

<400> 57
gtaagtggcc ccatcaccca ctgtggggac aggaaagcca agccagagag gcagggtcaa 60
tccagggtac ctctcagctg acaggaagaa gtgatggggg aaaggtcagg aggaggggga 120
catgggaaca ggggtaggcc agttccatgt ggctgatgca 160

<210> 58
<211> 514
<212> DNA
<213> human

<220>
<221> misc_feature
<222> (1)..(514)
<223> /note="Intron 8"

<400> 58
gtgagggtcc cagcaggggt agtcacaggc atctttcttc ccctccccct gcctcccttt 60
ccctgcctag aaaatgggct cttgtgcctg gcagctctag cttcctcagg tccatgcagt 120
ccctgatact gcaccccacc cctgaagtgt ccccgccccc agctaggcca gcctccccct 180
ccacccccat ggctagaggc ctcccctgca gaaaggatct gggcaccctt cctccagcct 240
aagcctcgcc tggcccccag gagtgtccaa gtcaatcata gggtcagtgt ggcagtgaga 300
aggaggtgcc tgtaggagag atgcagacct gagtggggat gccggagtgt gggagatcac 360
aggaggggat ggagaggtgg ggtgggggtt gagtacagga aagggtggga agctagggag 420
gtgtagagga gaggatatat gaaaggtgga cacccactat cccctgtctt ttctctttcc 480
ccacatggcc atcaccccct ccccaacatg ccag 514

<210> 59
<211> 72
<212> DNA
<213> human

<220>
<221> misc_feature
<222> (1)..(72)
<223> /note ="Exon 9"

<400> 59
acgtggctgg accccgaccc tgccaacgct ccctgtcggt gctcagcccc cgccaaccag 60
cccagaaccg ag 72

<210> 60
<211> 81
<212> DNA
<213> human

<220>
<221> misc_feature
<222> (1)..(81)
<223> /note="Intron 9"

<400> 60

gtactaccta ttctcaccct gcctaggatc tgtgcagagg ctgtcccacc cagttgctga 60
cagccctcct gttccttcta g 81

<210> 61
<211> 522
<212> DNA
<213> human

<220>
<221> misc_feature
<222> (1)..(522)
<223> /note="Exon 10"

<400> 61
agtccacccc ccttgccagc ggaccttcct cattccagcg ggctggctct gtgcgggatc 60
gtgtccacaa gttcacatct gattctccta tggctgctag gctccaggat ggcacacccc 120
aggctgccct aagtcccctg accccccgca aggctcctgg gcccctccct caccagcacc 180
acccctgcgt cctcctccag cggctcctcc tctcggggcc cagtgatacc tcctcccggt 240
tcagcaagga gcaacgagga gtagcccagc ccctggccca gcttcgaagc tgcccccagg 300
aggagggccc cagggggcgg ggcttggctg ctaggcccct tgaaaacaga gcaggggggc 360
ctgtggcacg ttcagaggag cctggtgccc cgctgcccgt ggccgtcggc actgccgagc 420
caggggacag tatgaagacc acattcacca tcgagatcaa ggacggccgt ggccaggcct 480
ccacaggccg ggtgctgctg cccacaggca accagagggc ag 522